# EUROPÄISCHE PATENTSCHRIFT

(11) **EP 1 719 814 B1**
(45) Veröffentlichungstag und Bekanntmachung des Hinweises auf die Patenterteilung: **16.03.2011**
(21) Anmeldenummer: 06112971.4
(22) Anmeldetag: 24.04.2006
(51) Int. Cl.: C12N 1/21, C12N 9/00, C12N 15/52, C12P 13/04, C12P 13/08

(54) **Verfahren zur Herstellung von L-Aminosäuren unter Verwendung von verbesserten Stämmen der Familie Enterobacteriaceae**
Process for the preparation of L-amino acids using strains of the family Enterobacteriaceae
Procédé de préparation de L-acides aminés utilisant des souches de la famille des Entérobactériacées

(30) Priorität: 03.05.2005 DE 102005020538
(43) Veröffentlichungstag der Anmeldung: 08.11.2006
(73) Patentinhaber: Evonik Degussa GmbH, 45128 Essen (DE)
(72) Erfinder: Dusch, Nicole, Dr., 33824 Werther (DE)

(56) Entgegenhaltungen:
- WO-A-03/008607
- WO-A-2004/087937
- US-A- 4 278 765
- GIMENEZ ROSA ET AL: "The gene yjcG, cotranscribed with the gene acs, encodes an acetate permease in Escherichia coli." JOURNAL OF BACTERIOLOGY, Bd. 185, Nr. 21, November 2003 (2003-11), Seiten 6448-6455, XP002389869 ISSN: 0021-9193
- DATABASE UniProt 1. Mai 2005 (2005-05-01), JIN Q ET AL: "Cation/ acetate symporter actP (Acetate transporter actP) (Acetate permease)" XP002389911 Database accession no. Q83P94
- DATABASE UniProt 1. Mai 2005 (2005-05-01), MC CLELLAND M ET AL: "Cation/ acetate symporter actP (Acetate transporter actP) (Acetate permease)" XP002389912 Database accession no. Q8ZKF8

## Beschreibung

Diese Erfindung betrifft ein Verfahren zur Herstellung von L-Threonin unter Verwendung von rekombinanten Mikroorganismen der Familie Enterobacteriaceae, in denen der offene Leserahmen (ORF) mit der Bezeichnung yjcG verstärkt, insbesondere überexprimiert wird, und diese Mikroorganismen.

### Stand der Technik

L-Aminosäuren, insbesondere L-Threonin, finden in der Humanmedizin und in der pharmazeutischen Industrie, in der Lebensmittelindustrie und ganz besonders in der Tierernährung Anwendung.

Es ist bekannt, dass L-Aminosäuren durch Fermentation von Stämmen der Enterobacteriaceae, insbesondere Escherichia coli (E. coli) und Serratia marcescens, hergestellt werden. Wegen der großen Bedeutung wird ständig an der Verbesserung der Herstellverfahren gearbeitet. Verfahrensverbesserungen können fermentationstechnische Maßnahmen, wie z.B. Rührung und Versorgung mit Sauerstoff, oder die Zusammensetzung der Nährmedien wie z.B. die Zuckerkonzentration während der Fermentation, oder die Aufarbeitung zur Produktform, durch z.B. Ionenaustauschchromatographie, oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Threonin-Analogon α-Amino-β-Hydroxyvaleriansäure (AHV) oder auxotroph für regulatorisch bedeutsame Metabolite sind und L-Aminosäuren wie z.B. L-Threonin produzieren.

Seit einigen Jahren werden ebenfalls Methoden der rekombinanten DNA-Technik zur Stammverbesserung L-Aminosäuren produzierender Stämme der Familie Enterobacteriaceae eingesetzt, indem man einzelne Aminosäure-Biosynthesegene amplifiziert und die Auswirkung auf die Produktion untersucht. Zusammenfassende Informationen zur Zell- und Molekularbiologie von Escherichia coli und Salmonella sind in Neidhardt (ed): Escherichia coli and Salmonella, Cellular and Molecular Biology, 2nd edition, ASM Press, Washington, D.C., USA, (1996) zu finden.

### Aufgabe der Erfindung

Die Erfinder haben sich die Aufgabe gestellt, neue Maßnahmen zur verbesserten fermentativen Herstellung von L-Threonin bereitzustellen.

### Beschreibung der Erfindung

Als Ausgangspunkt für den Vergleich dienen jeweils die für den yjcG-ORF nicht rekombinanten Mikroorganismen, die keinen verstärkten yjcG-ORF enthalten und an denen die Maßnahmen der Erfindung durchgeführt werden.

Zu diesen Mikroorganismen gehören insbesondere Mikroorganismen der Familie Enterobacteriaceae, in denen ein Polynukleotid verstärkt wird, das für ein Polypeptid kodiert, dessen Aminosäuresequenz zu mindestens 90%, insbesondere zu mindestens 95%, bevorzugt zu mindestens 98%

Gegenstand der Erfindung sind L-Threonin produzierende und ausscheidende rekombinante Mikroorganismen der Familie Enterobacteriaceae, in denen ein yjcG-ORF überexprimiert wird, der für ein Polypeptid kodiert, dessen Aminosäuresequenz zu mindestens 90% identisch ist mit einer Aminosäuresequenz, ausgewählt aus der Gruppe SEQ ID No. 2, SEQ ID No. 4 und SEQ ID No. 6 und welches die Aktivität der Acetat Permease aufweist, wobei die Überexpression durch Erhöhung der Kopienzahl des yjcG-ORF's und/oder Verwendung eines bereits starken Promotors erreicht wird und in denen man zusätzlich gleichzeitig
a) mindestens ein Gen des für die Aspartatkinase, die Homoserin-Dehydrogenase, die Homoserinkinase und die Threoninsynthase kodierenden thrABC-Operons, und gegebenenenfalls eines oder mehrere der gene, ausgewählt aus der Gruppe
b) das für die Pyruvat-Carboxylase kodierende pyc-Gen von Corynebacterium glutamicum,
c) das für die Phosphoenolpyruvat-Synthase kodierende pps-Gen,
d) das für die Phosphoenolpyruvat-Carboxylase kodierende ppc-Gen,
e) die für die Untereinheiten der Pryridin-Transhydrogenase kodierenden Gene pntA und pntB,
f) das für das Threoninresistenz vermittelnde Protein kodierende Gen rhtC,
g) das für das Threonin-Export-Carrier-Protein kodierende thrE-Gen aus Corynebacterium glutamicum,
h) das für die Glutamat-Dehydrogenase kodierende gdhA-Gen,
i) das für die Phosphohistidin-Protein-Hexose-Phosphotransferase kodierende ptsH-Gen,
j) das für das Enzym I des Phosphotransferase-Systems kodierende ptsI-Gen,
k) das für die Glucose-spezifische IIA Komponente kodierende crr-Gen,
l) das für die Glucose-spezifische IIBC Komponente kodierende ptsG-Gen,
m) das für die Cystein-Synthase A kodierende cysK-Gen,
n) das für den Regulator des cys-Regulons kodierende cysB.-Gen,
o) das für das Flavoprotein der NADPH-Sulfit-Reduktase kodierende cysJ-Gen,
p) das für das Hämoprotein der NADPH-Sulfit-Reduktase kodierende cysI-Gen,
q) das für die Adenylylsulfat-Reduktase kodierende cysH-Gen,
r) das für die Decarboxylase Untereinheit der 2-Ketoglutarat Dehydrogenase kodierende sucA-Gen,
s) das für die Dihydrolipoyltranssuccinase E2 Untereinheit der 2-Ketoglutarat Dehydrogenase kodierende sucB-Gen,
t) das für die-β-Untereinheit der Succinyl-CoA Synthetase kodierende sucC-Gen,
u) das für die α-Untereinheit der Succinyl-CoA Synthetase kodierende sucD-Gen, und
v) das Genprodukt des offenen Leserahmens (ORF) yibD von Escherichia colI
w)
überexprimiert.
oder mindestens 99%, besonders bevorzugt zu 99,8% und ganz besonders bevorzugt zu 100% identisch ist mit einer Aminosäuresequenz, ausgewählt aus der Gruppe SEQ ID No. 2, SEQ ID No. 4 und SEQ ID No. 6.

Die genannten Mikroorganismen enthalten verstärkte oder überexprimierte Polynukleotide, ausgewählt aus der Gruppe:
a) Polynukleotid mit einer Nukleotidsequenz, ausgewählt aus SEQ ID No. 1, SEQ ID No. 3 oder SEQ ID No. 5 und den dazu komplementären Nukleotidsequenzen;
b) Polynukleotid mit einer Nukleotidsequenz, die der SEQ ID No. 1, SEQ ID No. 3 oder SEQ ID No. 5 im Rahmen der Degeneration des genetischen Codes entspricht;
c) Polynukleotidsequenz mit einer Sequenz, die mit der zur Sequenz SEQ ID No. 1, SEQ ID No. 3 oder SEQ ID No. 5 komplementären Sequenz unter stringenten Bedingungen hybridisiert, wobei die stringenten Bedingungen bevorzugt durch einen Waschschritt erreicht werden, in dem sich die Temperatur über einen Bereich von 64°C bis 68°C und die Salzkonzentration des Puffers über einen Bereich von 2xSSC bis 0,1xSSC erstrecken;
d) Polynukleotid mit einer Sequenz SEQ ID No. 1, SEQ ID No. 3 oder SEQ ID No. 5, die funktionsneutrale Sinnmutanten enthält,
wobei die Polynukleotide bevorzugt für eine Acetat Permease kodieren.

Gegenstand der Erfindung ist ebenso ein Verfahren zur fermentativen Herstellung von L-Threonin, unter Verwendung von rekombinanten Mikroorganismen der Familie Enterobacteriaceae, die insbesondere bereits L-Treonin produzieren, und in denen mindestens der offene Leserahmen (ORF) mit der Bezeichnung yjcG oder für dessen Genprodukt kodierende Nukleotidsequenzen verstärkt wird bzw. werden.

Es werden die beschriebenen Mikroorganismen eingesetzt.

Der Begriff "Verstärkung" beschreibt in diesem Zusammenhang die Erhöhung der intrazellulären Aktivität oder Konzentration eines oder mehrerer Enzyme oder Proteine in einem Mikroorganismus, die durch die entsprechende DNA kodiert werden, indem man beispielsweise die Kopienzahl des Gens bzw. der Gene, des ORFs bzw. der ORFs um mindestens eine (1) Kopie erhöht, einen starken Promotor funktionell mit dem Gen verknüpft oder ein Gen oder Allel oder ORF verwendet, das für ein entsprechendes Enzym bzw. Protein mit einer hohen Aktivität kodiert und gegebenenfalls diese Maßnahmen kombiniert.

Als offener Leserahmen (ORF, open reading frame) wird ein Abschnitt einer Nukleotidsequenz bezeichnet, der für ein Protein beziehungsweise Polypeptid oder Ribonukleinsäure kodiert oder kodieren kann, dem (der) nach dem Stand der Technik keine Funktion zugeordnet werden kann. Nach Zuordnung einer Funktion zu dem betreffenden Abschnitt der Nukleotidsequenz wird im Allgemeinen von einem Gen gesprochen. Unter Allelen versteht man im Allgemeinen alternative Formen eines gegebenen Gens. Die Formen zeichnen sich durch Unterschiede in der Nukleotidsequenz aus.

Als Genprodukt bezeichnet man im Allgemeinen das von einer Nukleotidsequenz, d.h. einem ORF, einem Gen oder einem Allel kodierte Protein oder die kodierte Ribonukleinsäure.

Durch die Maßnahmen der Verstärkung, insbesondere Überexpression, wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des Wildtyp-Proteins beziehungsweise auf die Aktivität oder Konzentration des Proteins im für das entsprechende Enzym bzw. Protein nicht rekombinanten Mikroorganismus oder Elternstamm erhöht. Als nicht rekombinanter Mikroorganismus oder Elternstamm (parent strain) wird der Mikroorganismus verstanden, an dem die erfinderischen Maßnahmen durchgeführt werden.

offenbart wird, ein Verfahren zur Herstellung von L-Aminosäuren durch Fermentation von rekombinanten Mikroorganismen der Familie Enterobacteriaceae, dadurch gekennzeichnet, dass man
a) die L-Threonin produzierenden Mikroorganismen, in denen man den offenen Leserahmen yjcG oder für das Genprodukt kodierende Nukleotidsequenzen oder Allele verstärkt, insbesondere überexprimiert, in einem Medium kultiviert unter Bedingungen, bei denen die L-Threonin im Medium oder in den Zellen angereichert wird, und,
b) L-Threonin isoliert, wobei gegebenenfalls Bestandteile der Fermentationsbrühe und/oder die Biomasse in ihrer Gesamtheit oder Anteilen (≥ 0 bis 100 %) im isolierten Produkt verbleiben oder vollständig entfernt werden.

Die insbesondere rekombinanten Mikroorganismen mit einem verstärkten oder überexprimierten offenen Leserahmen (ORF) mit der Bezeichnung yjcG können L-Aminosäuren aus Glucose, Saccharose, Laktose, Fructose, Maltose, Melasse, gegebenenfalls Stärke, gegebenenfalls Cellulose oder aus Glycerin und Ethanol herstellen. Es handelt sich um Vertreter der Familie Enterobacteriaceae, ausgewählt aus den Gattungen Escherichia, Erwinia, Providencia und Serratia. Die Gattungen Escherichia und Serratia werden bevorzugt. Bei der Gattung Escherichia ist insbesondere die Art Escherichia coli und bei der Gattung Serratia insbesondere die Art Serratia marcescens zu nennen.

Rekombinante Mikroorganismen werden im Allgemeinen durch Transformation, Transduktion oder Konjugation, oder einer Kombination dieser Methoden, mit einem Vektor erzeugt, der den gewünschten ORF, das gewünschte Gen, ein Allel dieses ORFs oder Gens oder Teile davon und/oder einen die Exprimierung des ORFs oder Gens verstärkenden Promotor enthält. Bei diesem Promotor kann es sich um den durch verstärkende Mutation aus der eigenen, upstream des Gens oder ORFs befindlichen regulatorischen Sequenz hervorgegangenen Promotor handeln, oder es wurde ein effizienter Promotor mit dem Gen oder ORF fusioniert.

Als Elternstamm geeignete, insbesondere L-Threonin produzierende Stämme der Gattung Escherichia, insbesondere der Art Escherichia coli sind beispielsweise zu nennen:
- Escherichia coli H4581 (EP 0 301 572)
- Escherichia coli KY10935 (Bioscience Biotechnology and Biochemistry 61(11):1877-1882 (1997)
- Escherichia coli VNIIgenetika MG442 (US-A-4278,765)
- Escherichia coli VNIIgenetika M1 (US-A-4,321,325)
- Escherichia coli VNIIgenetika 472T23 (US-A-5,631,157)
- Escherichia coli BKIIM B-3996 (US-A-5,175,107)
- Escherichia coli kat 13 (WO 98/04715)
- Escherichia coli KCCM-10132 (WO 00/09660)

Als Elternstamm geeignete L-Threonin produzierende Stämme der Gattung Serratia, insbesondere der Art Serratia marcescens sind beispielsweise zu nennen:
- Serratia marcescens HNr21 (Applied and Environmental Microbiology 38 (6) : 1045-1051 (1979))
- Serratia marcescens TLr156 (Gene 57(2-3): 151-158 (1987))
- Serratia marcescens T-2000 (Applied Biochemistry and Biotechnology 37(3): 255-265 (1992))

L-Threonin produzierende Stämme aus der Familie der Enterobacteriaceae besitzen bevorzugt, unter anderen, ein oder mehrere der genetischen bzw. phänotypischen Merkmale ausgewählt aus der Gruppe: Resistenz gegen α-Amino-β-Hydroxyvaleriansäure, Resistenz gegen Thialysin, Resistenz gegen Ethionin, Resistenz gegen α-Methylserin, Resistenz gegen Diaminobernsteinsäure, Resistenz gegen α-Aminobuttersäure, Resistenz gegen Borrelidin, Resistenz gegen Cyclopentan-Carboxylsäure, Resistenz gegen Rifampicin, Resistenz gegen Valin-Analoga wie beispielsweise Valinhydroxamat, Resistenz gegen Purinanaloga wie beispielsweise 6-Dimethylaminopurin, Bedürftigkeit für L-Methionin, gegebenenfalls partielle und kompensierbare Bedürftigkeit für L-Isoleucin, Bedürftigkeit für meso-Diaminopimelinsäure, Auxotrophie bezüglich Threonin-haltiger Dipeptide, Resistenz gegen L-Threonin, Resistenz gegen Threonin-Raffinat, Resistenz gegen L-Homoserin, Resistenz gegen L-Lysin, Resistenz gegen L-Methionin, Resistenz gegen L-Glutaminsäure, Resistenz gegen L-Aspartat, Resistenz gegen L-Leucin, Resistenz gegen L-Phenylalanin, Resistenz gegen L-Serin, Resistenz gegen L-Cystein, Resistenz gegen L-Valin, Empfindlichkeit gegenüber Fluoropyruvat, defekte Threonin-Dehydrogenase, gegebenenfalls Fähigkeit zur Saccharose-Verwertung, Verstärkung des Threonin-Operons, Verstärkung der Homoserin-Dehydrogenase I-Aspartatkinase I bevorzugt der feed back resistenten Form, Verstärkung der Homoserinkinase, Verstärkung der Threoninsynthase, Verstärkung der Aspartatkinase, gegebenenfalls der feed back resistenten Form, Verstärkung der Aspartatsemialdehyd-Dehydrogenase, Verstärkung der Phosphoenolpyruvat-Carboxylase, gegebenenfalls der feed back resistenten Form, Verstärkung der Phosphoenolpyruvat-Synthase, Verstärkung der Transhydrogenase, Verstärkung des RhtB-Genproduktes, Verstärkung des RhtC-Genproduktes, Verstärkung des YfiK-Genproduktes, Verstärkung einer Pyruvat-Carboxylase, und Abschwächung der Essigsäurebildung.

Es wurde gefunden, dass Mikroorganismen der Familie Enterobacteriaceae nach Überexpression des Gens oder offenen Leserahmens (ORF) yjcG, oder dessen Allelen, eine verbesserte Fähigkeit zur Bildung und Anreicherung von L-Threonin, zeigen.

Die Nukleotidsequenzen der Gene oder offenen Leserahmen (ORF) von Escherichia coli gehören zum Stand der Technik und können der von Blattner et al. (Science 277: 1453-1462 (1997)) publizierten Genomsequenz von Escherichia coli entnommen werden. Es ist bekannt, dass durch wirtseigene Enzyme (Methionin-Aminipeptidase) die N-terminale Aminosäure Methionin abgespalten werden kann.

Aus den ebenfalls zur Familie Enterobacteriaceae gehörenden Salmonella typhimurium (Accession No.: NC_003197 (Region 4511508-4513157)) und Shigella flexneri (Accession No.: NC_004337 (Region 4293843-4295492)) ist die Nukleotidsequenz für den yjcG-ORF ebenso bekannt.

Der yjcG-ORF von Escherichia coli K12 wird unter anderem durch folgende Angaben beschrieben:

Gimenez et al. (Journal of Microbiology 185(21): 6448-55 (2003)) beschreiben den offenen Leserahmen yjcG aus Escherichia coli kodierend für ein Protein mit der Funktion einer Acetat Permease aus der Solute:Sodium Symporter (SSS) Familie und schlagen den Gennamen actP vor; das Gen wird kotranskribiert mit acs, welches für eine Acetyl Coenzym A Synthetase kodiert; die Permease ist hoch spezifisch für kurzkettigen aliphatische Monocarboxylate und transportiert neben Acetat auch Glycolat in zum Wachstum ausreichender Menge sowie kleine Carboxylate, wie z.B. Propionat.

Accession No.: NC000913 (Region 4281276-4282925) Alternativer Genname: b4067, actP

Die Nukleinsäuresequenzen können den Datenbanken des National Center for Biotechnology Information (NCBI) der National Library of Medicine (Bethesda, MD, USA), der Nukleotidsequenz-Datenbank der European Molecular Biologies Laboratories (EMBL, Heidelberg, Deutschland bzw. Cambridge, UK) oder der DNA Datenbank von Japan (DDBJ, Mishima, Japan) entnommen werden.

Der besseren Übersichtlichkeit halber sind die bekannte Sequenz zum yjcG-ORF von Escherichia coli unter der SEQ ID No. 1 und die bekannten Sequenzen zum yjcG-ORF von Salmonella typhimurium bzw. Shigella flexneri unter der SEQ ID No. 3 bzw. SEQ ID No. 5 dargestellt. Die von diesen Leserahmen kodierten Proteine sind als SEQ ID No. 2, SEQ ID No. 4 und SEQ ID No. 6 dargestellt.

Die in den angegebenen Textstellen beschriebenen offenen Leserahmen können erfindungsgemäß verwendet werden. Weiterhin können Allele der Gene oder offene Leserahmen verwendet werden, die sich aus der Degeneriertheit des genetischen Codes oder durch funktionsneutrale Sinnmutationen ("sense mutations") ergeben. Die Verwendung endogener Gene bzw. endogener offener Leserahmen wird bevorzugt.

Unter "endogenen Genen" oder "endogenen Nukleotidsequenzen" versteht man die in der Population einer Art vorhandenen Gene oder offene Leserahmen oder Allele beziehungsweise Nukleotidsequenzen.

Zu den Allelen des yjcG-ORFs, welche funktionsneutrale Sinnmutationen enthalten, zählen unter anderem solche, die zu höchstens 60 oder zu höchstens 50 oder zu höchstens 40 oder zu höchstens 30 oder zu höchstens 20, bevorzugt zu höchstens 10 oder zu höchstens 5, ganz besonders bevorzugt zu höchstens 3 oder zu höchstens 2 oder zu mindestens einem konservativen Aminosäureaustausch in dem von ihnen kodierten Protein führen.

Bei den aromatischen Aminosäuren spricht man von konservativen Austauschen wenn Phenylalanin, Tryptophan und Tyrosin gegeneinander ausgetauscht werden. Bei den hydrophoben Aminosäuren spricht man von konservativen Austauschen wenn Leucin, Isoleucin und Valin gegeneinander ausgetauscht werden. Bei den polaren Aminosäuren spricht man von konservativen Austauschen wenn Glutamin und Asparagin gegeneinander ausgetauscht werden. Bei den basischen Aminosäuren spricht man von konservativen Austauschen wenn Arginin, Lysin und Histidin gegeneinander ausgetauscht werden. Bei den sauren Aminosäuren spricht man von konservativen Austauschen wenn Asparaginsäure und Glutaminsäure gegeneinander ausgetauscht werden. Bei den Hydroxyl-Gruppen enthaltenden Aminosäuren spricht man von konservativen Austauschen wenn Serin und Threonin gegeneinander ausgetauscht werden.

In gleicher Weise können auch solche Nukleotidsequenzen verwendet werden, die für Varianten der genannten Proteine kodieren, die zusätzlich am N- oder C-Terminus eine Verlängerung oder Verkürzung um mindestens eine (1) Aminosäure enthalten. Diese Verlängerung oder Verkürzung beträgt nicht mehr als 60, 50, 40, 30, 20, 10, 5, 3 oder 2 Aminosäuren oder Aminosäurereste.

Zu den geeigneten Allelen zählen auch solche, die für Proteine kodieren, in denen mindestens eine (1) Aminosäure eingefügt (Insertion) oder entfernt (Deletion) ist. Die maximale Anzahl derartige als Indels bezeichneter Veränderungen kann 2, 3, 5, 10, 20 in keinem Falle aber mehr als 30 Aminosäuren betreffen.

Zu den geeigneten Allelen gehören ferner solche die durch Hybridisierung, insbesondere unter stringenten Bedingungen unter Verwendung von SEQ ID No. 1, SEQ ID No. 3 oder SEQ ID No. 5 oder Teilen davon insbesondere der Kodierregionen bzw. der dazu komplementären Sequenzen, erhältlich sind.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen Sonde und Zielsequenz, d. h. die mit der Sonde behandelten Polynukleotide, mindestens 80% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 80% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2x SSC oder 0,1x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz der eingesetzten Sonde bzw. zu den in SEQ ID No. 1, SEQ ID No. 3 oder SEQ ID No. 5 dargestellten Nukleotidsequenzen besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558) .

Zur Erzielung einer Verstärkung können beispielsweise die Expression der Gene oder offenen Leserahmen oder Allele oder die katalytischen Eigenschaften des Proteins erhöht werden. Gegebenenfalls können beide Maßnahmen kombiniert werden.

Zur Erzielung einer Überexpression kann beispielsweise die Kopienzahl der entsprechenden Gene oder offenen Leserahmen erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen L-Threonin-Produktion zu steigern, des weiteren vorteilhaft sein kann auch die Verwendung von Promotoren zur Genexpression, die eine andere zeitliche Genexpression ermöglicht. Auf der Ebene der translationalen Regulation der Genexpression ist es möglich, die Häufigkeit der Initiation (Anlagerung des Ribosoms an die m-RNA) oder die Geschwindigkeit der Elongation (Verlängerungsphase) zu erhöhen. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die ORFs, Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Methoden der Überexpression sind im Stand der Technik hinlänglich - beispielsweise bei Makrides et al. (Microbiological Reviews 60 (3), 512-538 (1996)) - beschrieben. Durch Verwendung von Vektoren wird die Kopienzahl um mindestens eine (1) Kopie erhöht. Als Vektoren können Plasmide wie beispielsweise in der US 5,538,873 beschrieben verwendet werden. Als Vektoren können ebenfalls Phagen, beispielsweise der Phage Mu, wie in der EP 0332448 beschrieben, oder der Phage lambda (λ) verwendet werden. Eine Erhöhung der Kopienzahl kann auch dadurch erzielt werden, dass eine weitere Kopie in eine weitere Stelle des Chromosoms - beispielsweise in die att-site des Phagen X (Yu und Court, Gene 223, 77-81 (1998)) - eingebaut wird. In der US 5,939,307 wird beschrieben, dass durch Einbau von Expressionskassetten oder Promotoren wie beispielsweise tac-Promotor, trp-Promotor, lpp-Promotor oder PL-Promotor und P_{R}-Promotor des Phagen λ beispielsweise stromaufwärts des chromosomalen Threoninoperons eine Erhöhung der Expression erzielt werden konnte. In gleicher Weise können die Promotoren des Phagen T7, die gear-box-Promotoren oder der nar-Promotor verwendet werden. Derartige Expressionskassetten oder Promotoren können auch verwendet werden um, wie in der EP 0 593 792 beschrieben, plasmidgebundene Gene zu überexprimieren. Durch Verwendung des lacI^{Q}-Allels lässt sich wiederum die Expression plasmidgebundener Gene kontrollieren (Glascock und Weickert, Gene 223, 221-231 (1998)). Es ist weiterhin möglich, dass die Aktivität der Promotoren durch Modifikation ihrer Sequenz mittels einem oder mehreren Nukleotidaustauschen, durch Insertion (en)und/oder Deletion(en) erhöht ist. Eine andere zeitliche Genexpression kann beispielsweise wie bei Walker et al. (Journal of Bacteriology 181: 1269-80 (1999)) beschrieben durch die Verwendung des Wachstumsphasen-abhängigen fis Promotors erreicht werden. Die Geschwindigkeit der Elongation wird durch die Codon-Verwendung beeinflusst, durch den Gebrauch von Codons für im Elternstamm (parent strain) häufig vorkommenden t-RNAs kann die Genexpression verstärkt werden.

Allgemeine Anleitungen hierzu findet der Fachmann unter anderem bei Chang und Cohen (Journal of Bacteriology 134: 1141-1156 (1978)), bei Hartley und Gregori (Gene 13: 347-353 (1981)), bei Amann und Brosius (Gene 40: 183-190 (1985)), bei de Broer et al. (Proceedings of the National Academy of Sciences of the United States of America 80: 21-25 (1983)), bei LaVallie et al. (BIO/TECHNOLOGY 11: 187-193 (1993)), in PCT/US97/13359, bei Llosa et al. (Plasmid 26: 222-224 (1991)), bei Quandt und Klipp (Gene 80: 161-169 (1989)), bei Hamilton et al. (Journal of Bacteriology 171: 4617-4622 (1989)), bei Jensen und Hammer (Biotechnology and Bioengineering 58: 191-195 (1998)) und in bekannten Lehrbüchern der Genetik und Molekularbiologie.

In Enterobacteriaceae replizierbare Plasmidvektoren wie z.B. von pACYC184 abgeleitete Kloniervektoren (Bartolome et al.; Gene 102: 75-78 (1991)), pTrc99A (Amann et al.; Gene 69: 301-315 (1988)) oder pSC101-Derivate (Vocke und Bastia; Proceedings of the National Academy of Sciences USA 80(21): 6557-6561 (1983)) können verwendet werden. In einem erfindungsgemäßen Verfahren kann man einen mit einem Plasmidvektor transformierten Stamm einsetzen, wobei der Plasmidvektor mindestens den yjcG-ORF, oder für dessen Genprodukt kodierende Nukleotidsequenzen oder Allele trägt.

Unter dem Begriff Transformation versteht man die Aufnahme einer isolierten Nukleinsäure durch einen Wirt (Mikroorganismus).

Es ist ebenfalls möglich, Mutationen, welche die Expression der jeweiligen Gene oder offenen Leserahmen betreffen, durch Sequenzaustausch (Hamilton et al.; Journal of Bacteriology 171: 4617-4622 (1989)), Konjugation oder Transduktion in verschiedene Stämme zu überführen.

Nähere Erläuterungen zu den Begriffen der Genetik und Molekularbiologie findet man in bekannten Lehrbüchern der Genetik und Molekularbiologie wie beispielsweise dem Lehrbuch von Birge (Bacterial and Bacteriophage Genetics, 4th ed., Springer Verlag, New York (USA), 2000) oder dem Lehrbuch von Berg, Tymoczko and Stryer (Biochemistry, 5th ed., Freeman and Company, New York (USA), 2002) oder dem Handbuch von Sambrook et al. (Molekular Cloning, A Laboratory Manual, (3-Volume Set), Cold Spring Harbor Laboratory Press, Cold Spring Harbor (USA), 2001).

Weiterhin kann es für die Produktion von L-Threonin mit Stämmen der Familie Enterobacteriaceae vorteilhaft sein, zusätzlich zur Verstärkung des offenen Leserahmens yjcG, ein oder mehrere Enzyme des bekannten Threonin-Biosyntheseweges oder Enzyme des anaplerotischen Stoffwechsels oder Enzyme für die Produktion von reduziertem Nicotinamid-Adenin-Dinukleotid-Phosphat oder Enzyme der Glykolyse oder PTS-Enzyme oder Enzyme des Schwefelstoffwechsels zu verstärken. Die Verwendung endogener Gene wird im Allgemeinen bevorzugt.

So können beispielsweise gleichzeitig eines oder mehrere der Gene, ausgewählt aus der Gruppe
- mindestens ein Gen des für die Aspartatkinase, die Homoserin-Dehydrogenase, die Homoserinkinase und die Threoninsynthase kodierenden thrABC-Operons (US-A-4,278,765),
- das für die Pyruvat-Carboxylase kodierende pyc-Gen von Corynebacterium glutamicum (WO 99/18228),
- das für die Phosphoenolpyruvat-Synthase kodierende pps-Gen (Molecular and General Genetics 231(2): 332-336 (1992); WO 97/08333),
- das für die Phosphoenolpyruvat-Carboxylase kodierende ppc-Gen (WO 02/064808),
- die für die Untereinheiten der Transhydrogenase kodierenden Gene pntA und pntB (European Journal of Biochemistry 158: 647-653 (1986); WO 95/11985),
- das für das Threoninresistenz vermittelnde Protein kodierende Gen rhtC (EP-A-1 013 765),
- das für das Threonin-Export-Carrier-Protein kodierende thrE-Gen von Corynebacterium glutamicum (WO 01/92545),
- das für die Glutamat-Dehydrogenase kodierende gdhA-Gen (Nucleic Acids Research 11: 5257-5266 (1983); Gene 23: 199-209 (1983); DE19907347),
- das für die Phosphohistidin-Protein-Hexose-Phosphotransferase des Phosphotransferase-Systems PTS kodierende ptsH-Gen des ptsHIcrr-Operons (WO 03/004674),
- das für das Enzym I des Phosphotransferase-Systems PTS kodierende ptsI-Gen des ptsHIcrr-Operons (WO 03/004674),
- das für die Glucose-spezifische IIA Komponente des Phosphotransferase-Systems PTS kodierende crr-Gen des ptsHIcrr-Operons (WO 03/004674),
- das für die Glucose-spezifische IIBC Komponente kodierende ptsG-Gen (WO 03/004670),
- das für die Cystein-Synthase A kodierende cysK-Gen (WO 03/006666),
- das für den Regulator des cys-Regulons kodierende cysB-Gen (WO 03/006666),
- das für das Flavoprotein der NADPH-Sulfit-Reduktase kodierende cysJ-Gen des cysJIH-Operons (WO 03/006666),
- das für das Hämoprotein der NADPH-Sulfit-Reduktase kodierende cysI-Gen des cysJIH-Operons (WO 03/006666),
- das für die Adenylylsulfat-Reduktase kodierende cysH-Gen des cysJIH-Operons (WO 03/006666),
- das für die Decarboxylase Untereinheit der 2-Ketoglutarat Dehydrogenase kodierende sucA-Gen des sucABCD-Operons (WO 03/008614),
- das für die Dihydrolipoyltranssuccinase E2 Untereinheit der 2-Ketoglutarat Dehydrogenase kodierende sucB-Gen des sucABCD-Operons (WO 03/008614),
- das für die β-Untereinheit der Succinyl-CoA Synthetase kodierende sucC-Gen des suc ABCD-Operons (WO 03/008615),
- das für die α-Untereinheit der Succinyl-CoA Synthetase kodierende sucD-Gen des sucABCD-Operons (WO 03/008615), und
- das Genprodukt des offenen Leserahmens (ORF) yibD von Escherichia coli (Accession Number AE000439 des National Center for Biotechnology Information (NCBI, Bethesda, MD, USA), DE102004005836.9),
verstärkt, insbesondere überexprimiert werden.

Weiterhin kann es für die Produktion von L-Threonin vorteilhaft sein, zusätzlich zur Verstärkung des offenen Leserahmens yjcG, eines oder mehrere der Gene ausgewählt aus der Gruppe
- das für die Threonin-Dehydrogenase kodierende tdh-Gen (Journal of Bacteriology 169: 4716-4721 (1987)),
- das für die Malat-Dehydrogenase (E.C. 1.1.1.37) kodierende mdh-Gen (Archives in Microbiology 149: 36-42 (1987)),
- das Genprodukt des offenen Leserahmens (ORF) yjfA von Escherichia coli (Accession Number AAC77180 des National Center for Biotechnology Information (NCBI, Bethesda, MD, USA), WO 02/29080),
- das Genprodukt des offenen Leserahmens (ORF) ytfP von Escherichia coli (Accession Number AAC77179 des National Center for Biotechnology Information (NCBI, Bethesda, MD, USA), WO 02/29080),
- das für das Enzym Phosphoenolpyruvat-Carboxykinase kodierende pckA-Gen (WO 02/29080),
- das für die Pyruvat-Oxidase kodierende poxB-Gen (WO 02/36797),
- das für den DgsA-Regulator des Phosphotransferase-Systems kodierende dgsA-Gen (WO 02/081721), das auch unter der Bezeichnung mlc-Gen bekannt ist,
- das für den Fructose-Repressor kodierende fruR-Gen (WO 02/081698), das auch unter der Bezeichnung cra-Gen bekannt ist,
- das für den Sigma³⁸-Faktor kodierende rpoS-Gen (WO 01/05939), das auch unter der Bezeichnung katF-Gen bekannt ist und
- das für die Aspartat Ammonium-Lyase kodierende aspA-Gen (WO 03/008603)
abzuschwächen, insbesondere auszuschalten oder die Expression zu verringern.

Der Begriff "Abschwächung" beschreibt in diesem Zusammenhang die Verringerung oder Ausschaltung der intrazellulären Aktivität oder Konzentration eines oder mehrerer Enzyme bzw. Proteine in einem Mikroorganismus, die durch die entsprechende DNA kodiert werden, indem man beispielsweise einen schwächeren Promotor als im für das entsprechende Enzym bzw. Protein nicht rekombinanten Mikroorganismnus oder Elternstamm oder ein Gen bzw. Allel verwendet, das für ein entsprechendes Enzym bzw. Protein mit einer niedrigen Aktivität kodiert bzw. das entsprechende Enzym bzw. Protein oder den offenen Leserahmen oder das Gen inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im für das entsprechende Enzym bzw. Protein nicht rekombinanten Mikroorganismus oder Elternstamm, herabgesenkt. Als nicht rekombinanter Mikroorganismus oder Elternstamm (parent strain) wird der Mikroorganismus verstanden, an dem die erfinderischen Maßnahmen durchgeführt werden.

Zur Erzielung einer Abschwächung können beispielsweise die Expression der Gene oder offenen Leserahmen oder die katalytischen Eigenschaften der Enzymproteine herabgesetzt bzw. ausgeschaltet werden. Gegebenenfalls können beide Maßnahmen kombiniert werden.

Die Verringerung der Genexpression kann durch geeignete Kulturführung, durch genetische Veränderung (Mutation) der Signalstrukturen der Genexpression oder auch durch Antisense-RNA Technik erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann unter anderem beispielsweise bei Jensen und Hammer (Biotechnology and Bioengineering 58: 191-195 (1998)), bei Carrier und Keasling (Biotechnology Progress 15: 58-64 (1999)), Franch und Gerdes (Current Opinion in Microbiology 3: 159-164 (2000)) und in bekannten Lehrbüchern der Genetik und Molekularbiologie wie beispielsweise dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Mutationen, die zu einer Veränderung beziehungsweise Herabsetzung der katalytischen Eigenschaften von Enzymproteinen führen, sind aus dem Stand der Technik bekannt. Als Beispiele seien die Arbeiten von Qiu und Goodman (Journal of Biological Chemistry 272: 8611-8617 (1997)), Yano et al. (Proceedings of the National Academy of Sciences of the United States of America 95: 5511-5515 (1998)), Wente und Schachmann (Journal of Biological Chemistry 266: 20833-20839 (1991)) genannt. Zusammenfassende Darstellungen können bekannten Lehrbüchern der Genetik und Molekularbiologie wie z.B. dem von Hagemann ("Allgemeine Genetik", Gustav Fischer Verlag, Stuttgart, 1986) entnommen werden.

Als Mutationen kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nicht-konservative Aminosäureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stop-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stop-Kodon im Kodierbereich, so führt dies ebenfalls zu einem vorzeitigen Abbruch der Translation. Deletionen von mindestens einem (1) oder mehreren Kodonen führen typischerweise ebenfalls zu einem vollständigen Ausfall der Enzymaktivität.

Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Geeignete Mutationen in den Genen können durch Gen- bzw. Allelaustausch in geeignete Stämme eingebaut werden.

Eine gebräuchliche Methode ist die von Hamilton et al. (Journal of Bacteriology 171: 4617-4622 (1989)) beschriebene Methode des Genaustauschs mit Hilfe eines konditional replizierenden pSC101-Derivates pMAK705. Andere im Stand der Technik beschriebene Methoden wie beispielsweise die von Martinez-Morales et al. (Journal of Bacteriology 181: 7143-7148 (1999)) oder die von Boyd et al. (Journal of Bacteriology 182: 842-847 (2000)) können gleichfalls benutzt werden.

Es ist ebenfalls möglich, Mutationen in den jeweiligen Genen oder Mutationen, die die Expression der jeweiligen Gene oder offenen Leserahmen betreffen, durch Konjugation oder Transduktion in verschiedene Stämme zu überführen.

Weiterhin kann es für die Produktion von L-Threonin vorteilhaft sein, zusätzlich zur Verstärkung des offenen Leserahmens yjcG, unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der Produkt-Ausbeute (gebildetes Produkt pro verbrauchter Kohlenstoff-Quelle) und der Produkt-Bildung (gebildetes Produkt pro Volumen und Zeit) oder auch anderer Prozess-Parameter und Kombinationen davon, wird um mindestens 0,5% mindestens 1%, mindestens 1,5% oder mindestens 2% bezogen auf den nicht rekombinanten Mikroorganismus oder Elternstamm bzw. den Fermentationsprozess unter Verwendung desselben verbessert.

Die erfindungsgemäß hergestellten Mikroorganismen können im batch - Verfahren (Satzkultivierung), im fed batch (Zulaufverfahren), im repeated fed batch-Verfahren (repetitives Zulaufverfahren) oder in einem kontinuierlichen Verfahren (DE102004028859.3 oder US5,763,230) kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten.

Als Kohlenstoffquelle können Zucker und Kohlehydrate wie z.B. Glucose, Saccharose, Laktose, Fructose, Maltose, Melasse, Stärke und gegebenenfalls Cellulose, Öle und Fette wie z.B. Sojaöl, Sonnenblumenöl, Erdnussöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden. Beispielsweise können Mischungen von Glucose und Fruktose im Verhältnis von ca. 1:1, wie sie in der EP 1 225 230 beschrieben sind, eingesetzt werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muss weiterhin Salze von Metallen enthalten, wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Die Fermentation wird im Allgemeinen bei einem pH-Wert von 5,5 bis 9,0, insbesondere 6,0 bis 8,0, durchgeführt. Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten, werden Sauerstoff oder Sauerstoffhaltige Gasmischungen wie z.B. Luft in die Kultur eingetragen. Die Temperatur der Kultur liegt normalerweise bei 25°C bis 45°C und vorzugsweise bei 30°C bis 40°C. Durch die Tätigkeit der Mikroorganismen kommt es zu einer Anreicherung der L-Aminosäure in der Kulturbrühe. Die Kultur wird solange fortgesetzt, bis sich ein Maximum an L-Threonin gebildet hat. Dieses Ziel wird normalerweise innerhalb von 10 Stunden bis 160 Stunden erreicht.

Aus der entnommenen Kulturbrühe können die L-Aminosäuren gewonnen, gesammelt oder konzentriert und gegebenenfalls gereinigt werden. Typische Methoden zur Reinigung der L-Aminosäuren sind die Ionenaustauschchromatographie und die Kristallisation. Hierdurch erhält man weitgehend reine L-Aminosäuren.

Es ist ebenfalls möglich aus der entnommenen Kulturbrühe (= Fermentationsbrühe) ein Produkt herzustellen, indem man die in der Kulturbrühe enthaltene Biomasse des Bakteriums vollständig (100%) oder nahezu vollständig d.h. mehr als oder größer als (>) 90% entfernt und die übrigen Bestandteile der Fermentationsbrühe weitgehend d.h. zu 30% - 100%, bevorzugt größer gleich (≥) 50%, ≥70% oder ≥90% oder auch vollständig (100%) im Produkt belässt.

Zur Entfernung oder Abtrennung der Biomasse werden Separationsmethoden wie beispielsweise Zentrifugation, Filtration, Dekantieren, Flockung oder eine Kombination hieraus eingesetzt.

Die erhaltene Brühe wird anschließend mit bekannten Methoden wie beispielsweise mit Hilfe eines Rotationsverdampfers, Dünnschichtverdampfers, Fallfilmverdampfers, durch Umkehrosmose, durch Nanofiltration oder einer Kombination hieraus eingedickt beziehungsweise konzentriert.

Diese aufkonzentrierte Brühe wird anschließend durch Methoden der Gefriertrocknung, der Sprühtrocknung, der Sprühgranulation oder durch anderweitige Verfahren zu einem vorzugsweise rieselfähigen, feinteiligen Pulver aufgearbeitet. Dieses rieselfähige, feinteilige Pulver kann dann wiederum durch geeignete Kompaktier- oder GranulierVerfahren in ein grobkörniges, gut rieselfähiges, lagerbares und weitgehend staubfreies Produkt überführt werden. Das Wasser wird hierbei insgesamt zu mehr als 90% entfernt, sodass der Wassergehalt im Produkt kleiner als 10%, kleiner als 5% oder kleiner als 3% beträgt.

Die Analyse von L-Aminosäuren kann durch Anionenaustauschchromatographie mit anschließender Ninhydrin Derivatisierung erfolgen, so wie bei Spackman et al. (Analytical Chemistry 30: 1190-1206 (1958)) beschrieben, oder sie kann durch reversed phase HPLC erfolgen, so wie bei Lindroth et al. (Analytical Chemistry 51: 1167-1174 (1979)) beschrieben.

Das erfindungsgemäße Verfahren dient zur fermentativen Herstellung von L-Threonin

Die vorliegende Erfindung wird im Folgenden anhand von Ausführungsbeispielen näher erläutert.

Verwendete Minimal- (M9) und Vollmedien (LB) für Escherichia coli sind von J.H. Miller (A Short Course in Bacterial Genetics (1992), Cold Spring Harbor Laboratory Press) beschrieben. Die Isolierung von Plasmid-DNA aus Escherichia coli sowie alle Techniken zur Restriktion, Ligation, Klenow- und alkalische Phosphatasebehandlung werden nach Sambrook et al. (Molecular Cloning - A Laboratory Manual (1989) Cold Spring Harbor Laboratory Press) durchgeführt. Die Transformation von Escherichia coli wird, wenn nicht anders beschrieben, nach Chung et al. (Proceedings of the National Academy of Sciences of the United States of America 86: 2172-2175 (1989)) durchgeführt.

Die Bebrütungstemperatur bei der Herstellung von Stämmen und Transformanten ist 37°C.

### Beispiel 1

### Konstruktion des Expressionsplasmides pMW218yjcG

Das yjcG-Gen aus E. coli K12 wird unter Anwendung der Polymerase-Kettenreaktion (PCR) sowie synthetischen Oligonukleotiden amplifiziert. Ausgehend von der Nukleotidsequenz des yjcG-Gens in E. coli K12 MG1655 (Accession Number NC000913 (Region 4281276-4282925), Blattner et al. (Science 277: 1453-1474 (1997)) werden PCR-Primer synthetisiert (MWG Biotech, Ebersberg, Deutschland). Die Sequenzen der Primer werden so modifiziert, dass Erkennungsstellen für Restriktionsenzyme entstehen. Für den yjcG-1-Primer wird die Erkennungssequenz für HindIII und für den yjcG-2-Primer die Erkennungssequenz für SacI gewählt, die in der unten dargestellten Nukleotidabfolge durch Unterstreichen markiert:
yjcG-1:
   5' - GATCAAGCTTATCCGGCCTACATTCG - 3' (SEQ ID No. 7)
yjcG-2:
   5' - GATCTAGAGCTCGATTAATGCGCGCGGCCTT - 3' (SEQ ID No. 8)

Die für die PCR eingesetzte chromosomale E. coli K12 MG1655 DNA wird nach Herstellerangaben mit "Qiagen Genomic-tips 100/G" (QIAGEN, Hilden, Deutschland) isoliert. Ein ca. 2084 bp großes DNA-Fragment kann mit den spezifischen Primern unter Standard-PCR-Bedingungen (Innis et al. (1990) PCR Protocols. A Guide to Methods and Applications, Academic Press) mit der Vent-DNA-Polymerase (New England Biolaps GmbH, Frankfurt, Deutschland) amplifiziert werden (SEQ ID No. 9).

Das amplifizierte yjcG-Fragment wird mit dem Vektor pCR-Blunt II-TOPO (Zero TOPO TA Cloning Kit, Invitrogen, Groningen, Niederlande) den Herstellerangaben entsprechend ligiert und in den E. coli Stamm TOP10 transformiert. Die Selektion Plasmid tragender Zellen erfolgt auf LB Agar, der mit 50 pg/ml Kanamycin versetzt ist. Nach der Plasmid DNA Isolierung wird der Vektor mit den Enzymen PvuI und HindIII/SacI gespalten und nach Überprüfung der Spaltung im 0,8%tigen Agarosegel mit pCRBluntyjcG bezeichnet.

Anschließend wird der Vektor pCRBluntyjcG mit den Enzymen HindIII und SacI gespalten und das yjcG-Fragment nach Auftrennung im 0,8%tigem Agarosegel aus dem Gel isoliert (QIAquick Gel Extraction Kit, QIAGEN, Hilden, Deutschland) und mit dem low-copy Vektor pMW218 (Nippon Gene, Toyama, Japan), der mit den Enzymen HindIII und SacI verdaut worden ist, ligiert. Der E. coli Stamm DH5α (Grant et al.; Proceedings of the National Academy of Sciences USA, 87 (1990) 4645-4649) wird mit dem Ligationsansatz transformiert und Plasmid tragende Zellen auf LB Agar, der mit 50 pg/ml Kanamycin versetzt ist, selektioniert.

Die erfolgreiche Klonierung kann nach der Plasmid DNA Isolierung durch die Kontrollspaltung mit den Enzymen EcoRI/SalI nachgewiesen werden.

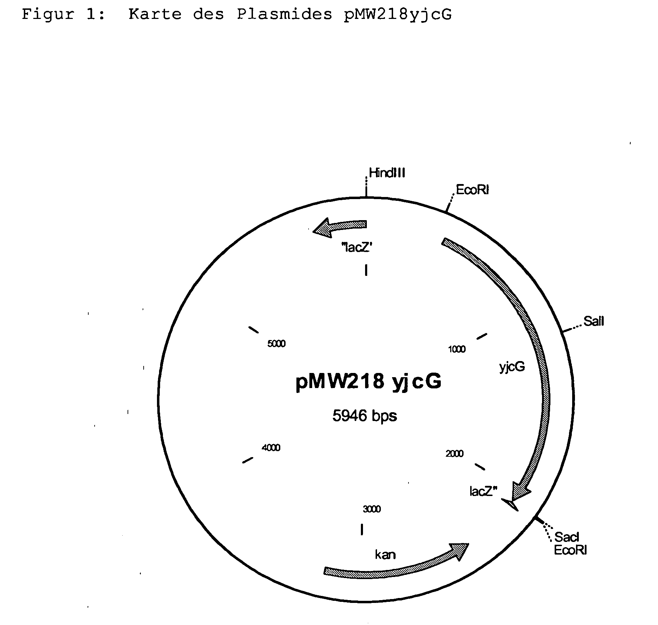

Das Plasmid wird als pMW218yjcG (Figur 1) bezeichnet.

### Beispiel 2

### Herstellung von L-Threonin mit dem Stamm MG442/pMW218yjcG

Der L-Threonin produzierende E. coli Stamm MG442 ist in der Patentschrift US-A- 4,278,765 beschrieben und bei der Russischen Nationalsammlung für industrielle Mikroorganismen (VKPM, Moskau, Russland) als CMIM B-1628 hinterlegt.

Der Stamm MG442 wird mit dem in Beispiel 1 beschriebenen Expressionsplasmid pMW218yjcG und mit dem Vektor pMW218 transformiert und auf LB Agar mit 50 µg/ml Kanamycin Plasmid tragende Zellen selektioniert. Auf diese Weise entstehen die Stämme MG442/pMW218yjcG und MG442/pMW218. Ausgewählte Einzelkolonien werden anschließend auf Minimalmedium mit der folgenden Zusammensetzung: 3,5 g/l Na₂HPO₄*2H₂O, 1,5 g/l KH₂PO₄, 1 g/l NH₄Cl, 0,1 g/l MgSO₄*7H₂O, 2 g/l Glucose, 20 g/l Agar, 50 mg/l Kanamycin, weiter vermehrt. Die Bildung von L-Threonin wird in batch Kulturen von 10 ml, die in 100 ml Erlenmeierkolben enthalten sind, überprüft. Dazu wird 10 ml Vorkulturmedium der folgenden Zusammensetzung: 2 g/l Hefeextrakt, 10 g/l (NH₄)₂SO₄, 1 g/l KH₂PO₄, 0,5 g/l MgSO₄*7H₂O, 15 g/l CaCO₃, 20 g/l Glucose, 50 mg/l Kanamycin, beimpft und für 16 Stunden bei 37°C und 180 rpm auf einem ESR Inkubator der Firma Kühner AG (Birsfelden, Schweiz) inkubiert. Je 250 µl dieser Vorkultur werden in 10 ml Produktionsmedium (25 g/l (NH₄)₂SO₄, 2 g/l KH₂PO₄, 1 g/l MgSO₄*7H₂O, 0,03 g/l FeSO₄*7H₂O, 0,018 g/l MnSO₄*1H₂O, 30 g/l CaCO₃, 20 g/l Glucose, 50 mg/l Kanamycin) überimpft und für 48 Stunden bei 37°C inkubiert. Nach der Inkubation wird die optische Dichte (OD) der Kultursuspension mit einem LP2W-Photometer der Firma Dr. Lange (Düsseldorf, Deutschland) bei einer Messwellenlänge von 660 nm bestimmt.

Anschließend wird die Konzentration an gebildetem L-Threonin im steril filtrierten Kulturüberstand mit einem Aminosäureanalysator der Firma Eppendorf-BioTronik (Hamburg, Deutschland) durch Ionenaustauschchromatographie und Nachsäulenreaktion mit Ninhydrindetektion bestimmt.

In Tabelle 1 ist das Ergebnis des Versuches dargestellt.

**Tabelle 1**

| Stamm | OD (660 nm) | L-Threonin g/l |
|---|---|---|
| MG442/pMW218 | 6,4 | 2,15 |
| MG442/pMW218yjcG | 5,5 | 2,5 |

Kurze Beschreibung der Figur:
Figur 1: Karte des das yjcG-Gen enthaltenden Plasmides pMW218yjcG

Längenangaben sind als ca.-Angaben aufzufassen. Die verwendeten Abkürzungen und Bezeichnungen haben folgende Bedeutung:
- kan: Gen, welches für die Kanamycinresistenz kodiert
- yjcG: Kodierregion des yjcG-Gens
- lacZ': Genfragment, welches für das α-Peptid der β-Galaktosidase kodiert

Die Abkürzungen für die Restriktionsenzyme haben folgende Bedeutung:
- EcoRI: Restriktionsendonuklease aus Escherichia coli
- HindIII: Restriktionsendonuklease aus Haemophilus influenzae
- SacI: Restriktionsendonuklease aus Streptomyces achromogenes
- SalI: Restriktionsendonuklease aus Streptomyces albus G

## Patentansprüche

1. L-Threonin produzierende und ausscheidende rekombinante Mikroorganismen der Familie Enterobacteriaceae, in denen ein yjcG-ORF überexprimiert wird, der für ein Polypeptid kodiert, dessen Aminosäuresequenz zu mindestens 90% identisch ist mit einer Aminosäuresequenz, ausgewählt aus der Gruppe SEQ ID No. 2, SEQ ID No. 4 und SEQ ID No. 6 und welches die Aktivität der Acetat Permease aufweist, wobei die Überexpression durch Erhöhung der Kopienzahl des yjcG-ORF's und/oder Verwendung eines bereits starken Promotors erreicht wird und in denen man zusätzlich gleichzeitig
a) mindestens ein Gen des für die Aspartatkinase, die Homoserin-Dehydrogenase, die Homoserinkinase und die Threoninsynthase kodierenden thrABC-Operons, und gegebenenfalls eines oder wehrere der gene, ausgewählt aus der gruppe
b) das für die Pyruvat-Carboxylase kodierende pyc-Gen von Corynebacterium glutamicum,
c) das für die Phosphoenolpyruvat-Synthase kodierende pps-Gen,
d) das für die Phosphoenolpyruvat-Carboxylase kodierende ppc-Gen,
e) die für dieUntereinheiten der Pryridin-Transhydrogenase kodierenden Gene pntA und pntB,
f) das für das Threoninresistenz vermittelnde Protein kodierende Gen rhtC,
g) das für das Threonin-Export-Carrier-Protein kodierende thrE-Gen aus Corynebacterium glutamicum,
h) das für die Glutamat-Dehydrogenase kodierende gdhA-Gen,
i) das für die Phosphohistidin-Protein-Hexose-Phosphotransferase kodierende ptsH-Gen,
j) das für das Enzym I des Phosphotransferase-Systems kodierende ptsI-Gen,
k) das für die Glucose-spezifische IIA Komponente kodierende crr-Gen,
l) das für die Glucose-spezifische IIBC Komponente kodierende ptsG-Gen,
m) das für die Cystein-Synthase A kodierende cysK-Gen,
n) das für den Regulator des cys-Regulons kodierende cysB-Gen,
o) das für das Flavoprotein der NADPH-Sulfit-Reduktase kodierende cysJ-Gen,
p) das für das Hämoprotein der NADPH-Sulfit-Reduktase kodierende cysI-Gen,
q) das für die Adenylylsulfat-Reduktase kodierende cysH-Gen,
r) das für die Decarboxylase Untereinheit der 2 Ketoglutarat Dehydrogenase kodierende sucA-Gen,
s) das für die Dihydrolipoyltranssuccinase E2 Untereinheit der 2-Ketoglutarat Dehydrogenase kodierende sucB-Gen,
t) dasfür die β-Untereinheit der Succinyl-CoA Synthetase kodierende sucC-Gen,
u) das für die α-Untereinheit der Succinyl-CoA Synthetase kodierende sucD-Gen, und
v) das Genprodukt des offenen Leserahmens (ORF) yibD von Escherichia coli
w)
überexprimiert.

2. Verfahren zur Herstellung von L-Threonin durch Fermentation von rekombinanten Mikroorganismen der Familie Enterobacteriaceae, **dadurch gekennzeichnet, dass** man
a) die L-Threonin produzierenden Mikroorganismen in denen ein yjcG-ORF überexprimiert wird, der für ein Polypeptid kodiert, dessen Aminosäuresequenz zu mindestens 90% identisch ist mit einer Aminosäuresequenz, ausgewählt aus der Gruppe SEQ ID No. 2, SEQ ID No. 4 und SEQ ID No. 6 und welches die Aktivität der Acetat Permease aufweist, wobei die Überexpression durch Erhöhung der Kopienzahl des yjcG-ORF's und/oder Verwendung eines bereits starken Promotors erreicht wird in einem Medium kultiviert unter Bedingungen, bei denen L-Threonin im Medium oder in den Zellen angereichert wird, und
b) die gewünschte L-Aminosäure isoliert, wobei Bestandteile der Fermentationsbrühe und/oder die Biomasse in ihrer Gesamtheit oder Anteilen (≥0 bis 100%) im isolierten Produkt verbleiben oder vollständig entfernt werden.

3. Verfahren gemäss Anspruch 2, **dadurch gekennzeichnet, dass** man zur Herstellung von L-Threonin Mikroorganismen fermentiert, in denen man zusätzlich gleichzeitig eines oder mehrere der Gene, ausgewählt aus der Gruppe:
a) mindestens ein Gen des für die Aspartatkinase, die Homoserin-Dehydrogenase, die Homoserinkinase und die Threoninsynthase kodierenden thrABC-Operons,
b) das für die Pyruvat-Carboxylase kodierende pyc-Gen von Corynebacterium glutamicum,
c) das für die Phosphoenolpyruvat-Synthase kodierende pps-Gen,
d) das für die Phosphoenolpyruvat-Carboxylase kodierende ppc-Gen,
e) die für die Untereinheiten der Pryridin-Transhydrogenase kodierenden Gene pntA und pntB,
f) das für das Threoninresistenz vermittelnde Protein kodierende Gen rhtC,
g) das für das Threonin-Export-Carrier-Protein kodierende thrE-Gen aus Corynebacterium glutamicum,
h) das für die Glutamat-Dehydrogenase kodierende gdhA-Gen,
i) das für die Phosphohistidin-Protein-Hexose-Phosphotransferase kodierende ptsH-Gen,
j) das für das Enzym I des Phosphotransferase-Systems kodierende ptsI-Gen,
k) das für die Glucose-spezifische IIA Komponente kodierende crr-Gen,
l) das für die Glucose-spezifische IIBC Komponente kodierende ptsG-Gen,
m) das für die Cystein-Synthase A kodierende cysK-Gen,
n) das für den Regulator des cys-Regulons kodierende cysB-Gen,
o) das für das Flavoprotein der NADPH-Sulfit-Reduktase kodierende cysJ-Gen,
p) das für das Hämoprotein der NADPH-Sulfit-Reduktase kodierende cysI-Gen,
q) das für die Adenylylsulfat-Reduktase kodierende cysH-Gen,
r) das für die Decarboxylase Untereinheit der 2-Ketoglutarat Dehydrogenase kodierende sucA-Gen,
s) das für die Dihydrolipoyltranssuccinase E2 Untereinheit der 2-Ketoglutarat Dehydrogenase kodierende sucB-Gen,
t) das für die β-Untereinheit der Succinyl-CoA Synthetase kodierende sucC-Gen,
u) das für die α-Untereinheit der Succinyl-CoA Synthetase kodierende sucD-Gen, und
v) das Genprodukt des offenen Leserahmens (ORF) yibD von Escherichia coli
überexpriment.

4. Verfahren gemäss den Ansprüchen 2 oder 3, **dadurch gekennzeichnet, dass** man zur Herstellung von L-Threonin Mikroorganismen fermentiert, in denen man zusätzlich gleichzeitig eines oder mehrere der Gene, ausgewählt aus der Gruppe:
a) das für die Threonin-Dehydrogenase kodierende tdh-Gen,
b) das für die Malat-Dehydrogenase kodierende mdh-Gen,
c) das Genprodukt des offenen Leserahmens (ORF) yjfA von Escherichia coli,
d) das Genprodukt des offenen Leserahmens (ORF) ytfP von Escherichia coli,
e) das für die Phosphoenolpyruvat-Carboxykinase kodierende pckA-Gen,
f) das für die Pyruvat-Oxidase kodierende poxB-Gen,
g) das für den DgsA-Regulator des Phosphotransferase-Systems kodierende dgsA-Gen,
h) das für den Fructose-Repressor kodierende fruR-Gen,
i) das für den Sigma³⁸-Faktor kodierende rpoS-Gen, und
j) das für die Aspartat Ammonium-Lyase kodierende aspA-Gen ausschaltet.

## Claims

1. Recombinant microorganisms of the Enterobacteriaceae family which produce and excrete L-threonine and in which an overexpressed yjcG-ORF encodes a polypeptide whose amino acid sequence is at least 90% identical to an amino acid sequence selected from the group SEQ ID No. 2, SEQ ID No. 4 and SEQ ID No. 6 and which has acetate permease activity, wherein the overexpression is achieved by increasing the copy number of the yjcG-ORF and/or using a promoter which is already strong, and in which
a) at least one gene of the thrABC operon encoding aspartate kinase, homoserine dehydrogenase, homoserine kinase and threonine synthase,
and optionally one or more of the genes selected from the group
b) the pyruvate carboxylase-encoding Corynebacterium glutamicum pyc gene,
c) the phosphoenolpyruvate synthase-encoding pps gene,
d) the phosphoenolpyruvate carboxylase-encoding ppc gene,
e) the pntA and pntB genes encoding the subunits of pyridine transhydrogenase,
f) the rhtC gene encoding the threonine resistance-mediating protein,
g) the threonine export carrier protein-encoding Corynebacterium glutamicum thrE gene,
h) the glutamate dehydrogenase-encoding gdhA gene,
i) the ptsH gene encoding the phosphohistidine protein hexose phosphotransferase,
j) the ptsI gene encoding enzyme I of the phosphotransferase system,
k) the crr gene encoding the glucose-specific IIA component,
l) the ptsG gene encoding the glucose-specific IIBC component,
m) the cysteine synthase A-encoding cysK gene,
n) the cysB gene encoding the regulator of the cys regulon,
o) the cysJ gene encoding the NADPH sulfite reductase flavoprotein,
p) the cysI gene encoding the NADPH sulfite reductase hemoprotein,
q) the adenylyl sulfate reductase-encoding cysH gene,
r) the sucA gene encoding the decarboxylase subunit of 2-ketoglutarate dehydrogenase,
s) the sucB gene encoding the dihydrolipoyltranssuccinase E2 subunit of 2-ketoglutarate dehydrogenase,
t) the sucC gene encoding the β-subunit of succinyl-CoA synthetase,
u) the sucD gene encoding the α-subunit of succinyl-CoA synthetase, and
v) the gene product of the Escherichia coli yibD open reading frame (ORF)
is/are additionally, at the same time, overexpressed.

2. Process for preparing L-threonine by fermenting recombinant microorganisms of the Enterobacteriaceae family, **characterized in that**
a) the L-threonine-producing microorganisms in which an overexpressed yjcG-ORF encodes a polypeptide whose amino acid sequence is at least 90% identical to an amino acid sequence selected from the group SEQ ID No. 2, SEQ ID No. 4 and SEQ ID No. 6 and which has acetate permease activity, wherein the overexpression is achieved by increasing the copy number of the yjcG-ORF and/or using a promoter which is already strong, are cultured in a medium under conditions under which L-threonine is enriched in the medium or in the cells, and
b) the desired L-amino acid is isolated, with constituents of the fermentation broth, and/or the biomass remaining in its/their entirety or in portions (from ≥ 0 to 100%) in the isolated product or being removed completely.

3. Process according to Claim 2, **characterized in that**, for the purpose of preparing L-threonine, microorganisms are fermented in which one or more of the genes selected from the group:
a) at least one gene of the thrABC operon encoding aspartate kinase, homoserine dehydrogenase, homoserine kinase and threonine synthase,
b) the pyruvate carboxylase-encoding Corynebacterium glutamicum pyc gene,
c) the phosphoenolpyruvate synthase-encoding pps gene,
d) the phosphoenolpyruvate carboxylase-encoding ppc gene,
e) the pntA and pntB genes encoding the subunits of pyridine transhydrogenase,
f) the rhtC gene encoding the threonine resistance-mediating protein,
g) the threonine export carrier protein-encoding Corynebacterium glutamicum thrE gene,
h) the glutamate dehydrogenase-encoding gdhA gene,
i) the ptsH gene encoding the phosphohistidine protein hexose phosphotransferase,
j) the ptsI gene encoding enzyme I of the phosphotransferase system,
k) the crr gene encoding the glucose-specific IIA component,
l) the ptsG gene encoding the glucose-specific IIBC component,
m) the cysteine synthase A-encoding cysK gene,
n) the cysB gene encoding the regulator of the cys regulon,
o) the cysJ gene encoding the NADPH sulfite reductase flavoprotein,
p) the cysI gene encoding the NADPH sulfite reductase hemoprotein,
q) the adenylyl sulfate reductase-encoding cysH gene,
r) the sucA gene encoding the decarboxylase subunit of 2-ketoglutarate dehydrogenase,
s) the sucB gene encoding the dihydrolipoyltranssuccinase E2 subunit of 2-ketoglutarate dehydrogenase,
t) the sucC gene encoding the β-subunit of succinyl-CoA synthetase,
u) the sucD gene encoding the α-subunit of succinyl-CoA synthetase, and
v) the gene product of the Escherichia coli yibD open reading frame (ORF)
is/are additionally, at the same time, overexpressed.

4. Process according to Claim 2 or 3, **characterized in that**, for the purpose of preparing L-threonine, microorganisms are fermented in which one or more of the genes selected from the group:
a) the threonine dehydrogenase-encoding tdh gene,
b) the malate dehydrogenase-encoding mdh gene,
c) the gene product of the Escherichia coli yjfA open reading frame (ORF),
d) the gene product of the Escherichia coli ytfP open reading frame (ORF),
e) the pckA gene encoding the phosphoenolpyruvate carboxykinase,
f) the pyruvate oxidase-encoding poxB gene,
g) the dgsA gene encoding the DgsA regulator of the phosphotransferase system,
h) the fruR gene encoding the fructose repressor,
i) the rpoS gene encoding the sigma³⁸ factor, and,
j) the aspartate ammonium lyase-encoding aspA gene,
is/are additionally, at the same time, eliminated.

## Revendications

1. Micro-organismes recombinants de la famille des *Enterobacteriaceae,* produisant et excrétant de la L-thréonine, dans lesquels est surexprimé un ORF yjcG qui code pour un polypeptide dont la séquence d'aminoacides est identique à raison d'au moins 90 % à une séquence d'aminoacides choisie dans le groupe constitué par SEQ ID n° 2, SEQ ID n° 4 et SEQ ID n° 6 et qui présente l'activité de l'acétate perméase, la surexpression étant atteinte par augmentation du nombre de copies de l'ORF yjcG et/ou l'utilisation d'un promoteur déjà fort et dans lesquels en outre on surexprime en même temps
a) au moins un gène de l'opéron thrABC codant pour l'aspartate kinase, l'homosérine déshydrogénase, l'homosérine kinase et la thréonine synthase,
et éventuellement un ou plusieurs gènes choisis dans le groupe constitué par
b) le gène pyc de *Corynebacterium glutamicum,* codant pour la pyruvate carboxylase,
c) le gène pps codant pour la phosphoénolpyruvate synthase,
d) le gène ppc pour la phosphoénolpyruvate carboxylase,
e) les gènes pntA et pntB codant pour les sous-unités de la pyridine transhydrogénase,
f) le gène rhtC codant pour la protéine conférant la résistance à la thréonine,
g) le gène thrE de *Corynebacterium glutamicum* codant pour la protéine export-carrier de thréonine,
h) le gène gdhA codant pour la glutamate déshydrogénase,
i) le gène ptsH codant pour phosphohistidine-protéine-hexose phosphotransférase,
j) le gène ptsI codant pour l'enzyme I du système phosphotransférase
k) le gène crr codant pour le composant IIA spécifique du glucose,
l) le gène ptsG codant pour le composant IIBC spécifique du glucose,
m) le gène cysK codant pour la cystéine synthase A,
n) le gène cysB codant pour le régulateur du régulon cys,
o) le gène cysJ codant pour la flavoprotéine de la NADPH-sulfite réductase,
p) le gène cysI codant pour l'hémoprotéine de la NADPH-sulfite réductase
q) le gène cysH codant pour l'adénylylsulfate réductase,
r) le gène sucA codant pour la sous-unité décarboxylase de la 2-cétoglutarate déshydrogénase,
s) le gène sucB codant pour la sous-unité dihydrolipoyltrans-succinase E2 de la 2-cétoglutarate déshydrogénase,
t) le gène sucC codant pour la sous-unité β Q de la succinyl-CoA synthétase,
u) le gène sucD codant pour la sous-unité α de la succinyl-CoA synthétase,
v) le produit génique du cadre de lecture ouvert (ORF) yibD *d'Escherichia coli.*

2. Procédé pour la production de L-thréonine par culture par fermentation de micro-organismes recombinant appartenant à la famille des *Enterobacteriaceae,* **caractérisé en ce que**
a) on cultive dans un milieu des micro-organismes produisant de la L-thréonine, dans lesquels est surexprimé un ORF yjcG qui code pour un polypeptide dont la séquence d'aminoacides est identique à raison d'au moins 90 % à une séquence d'aminoacides choisie dans le groupe constitué par SEQ ID n° 2, SEQ ID n° 4 et SEQ ID n° 6 et qui présente l'activité de l'acétate perméase, la surexpression étant atteinte par augmentation du nombre de copies de l'ORF yjcG et/ou l'utilisation d'un promoteur déjà fort, dans des conditions dans lesquelles la L-thréonine est accumulée dans le milieu ou dans les cellules, et
b) on isole le L-aminoacide recherché, les composants du bouillon de fermentation et/ou la biomasse restant en leur totalité ou en parties (≥ 0 à 100 %) dans le produit isolé ou étant totalement éliminés.

3. Procédé selon la revendication 2, **caractérisé en ce que** pour la production de L-thréonine on cultive par fermentation des micro-organismes dans lesquels dans lesquels en outre on surexprime en même temps un ou plusieurs gènes, choisis dans le groupe constitué par
a) au moins un gène de l'opéron thrABC codant pour l'aspartate kinase, l'homosérine déshydrogénase, l'homosérine kinase et la thréonine synthase,
b) le gène pyc de *Corynebacterium glutamicum,* codant pour la pyruvate carboxylase,
c) le gène pps codant pour la phosphoénolpyruvate synthase,
d) le gène ppc pour la phosphoénolpyruvate carboxylase,
e) les gènes pntA et pntB codant pour les sous-unités de la pyridine transhydrogénase,
f) le gène rhtC codant pour la protéine conférant la résistance à la thréonine,
g) le gène thrE de *Corynebacterium glutamicum* codant pour la protéine export-carrier de thréonine,
h) le gène gdhA codant pour la glutamate déshydrogénase,
i) le gène ptsH codant pour phosphohistidine-protéine-hexose phosphotransférase,
j) le gène ptsI codant pour l'enzyme I du système phosphotransférase
k) le gène crr codant pour le composant IIA spécifique du glucose,
l) le gène ptsG codant pour le composant IIBC spécifique du glucose,
m) le gène cysK codant pour la cystéine synthase A,
n) le gène cysB codant pour le régulateur du régulon cys,
o) le gène cysJ codant pour la flavoprotéine de la NADPH-sulfite réductase,
p) le gène cysI codant pour l'hémoprotéine de la NADPH-sulfite réductase
q) le gène cysH codant pour l'adénylylsulfate réductase,
r) le gène sucA codant pour la sous-unité décarboxylase de la 2-cétoglutarate déshydrogénase,
s) le gène sucB codant pour la sous-unité dihydrolipoyltrans-succinase E2 de la 2-cétoglutarate déshydrogénase,
t) le gène sucC codant pour la sous-unité β de la succinyl-CoA synthétase,
u) le gène sucD codant pour la sous-unité α de la succinyl-CoA synthétase,
v) le produit génique du cadre de lecture ouvert (ORF) yibD *d'Escherichia coli.*

4. Procédé selon la revendication 2 ou 3, **caractérisé en ce que** pour la production de L-thréonine on cultive par fermentation des micro-organismes dans lesquels dans lesquels en outre on inactive en même temps un ou plusieurs gènes, choisis dans le groupe constitué par
a) le gène tdh codant pour la thréonine déshydrogénase,
b) le gène mdh codant pour la malate déshydrogénase,
c) le produit génique du cadre de lecture ouvert (ORF) yjfA *d' Escherichia coli,*
d) le produit génique du cadre de lecture ouvert (ORF) ytfP *d'Escherichia coli,*
e) le gène pckA codant pour la phosphoénolpyruvate carboxykinase,
f) le gène poxB codant pour la pyruvate oxydase,
g) le gène dgsA codant pour le régulateur DgsA du système phosphotransférase,
h) le gène fruR codant pour le répresseur de fructose,
i) le gène rpoS codant pour le facteur Sigma³⁸, et
j) le gène AspA codant pour l'aspartate ammonium-lyase.


### SEQUENCE LISTING

<110> Degussa AG
<120> Verfahren zur Herstellung von L-Aminosäuren unter Verwendung von verbesserten Stämmen der Familie Enterobacteriaceae
<130> 050061BT
<160> 10
<170> PatentIn version 3.3
<210> 1
   <211> 1650
   <212> DNA
   <213> Escherichia coli
<220>
   <221> CDS ,
   <222> (1)..(1650)
   <223> yjcG Kodierregion
<400> 1
   atg aaa aga gtt ctg acg gcg ctt gcc gcc aca ctc cct ttc gca gct       48
   Met Lys Arg Val Leu Thr Ala Leu Ala Ala Thr Leu Pro Phe Ala Ala
   1               5                   10                  15
   aac gcc gcg gat gct att agc ggg gcc gta gag cgc cag cca acg aac       96
   Asn Ala Ala Asp Ala Ile Ser Gly Ala Val Glu Arg Gln Pro Thr Asn
               20                  25                  30
   tgg cag gcg att att atg ttc ctg att ttc gtc gtg ttt acg ctc ggc      144
   Trp Gln Ala Ile Ile Met Phe Leu Ile Phe Val Val Phe Thr Leu Gly
           35                  40                  45
   att acc tac tgg gca tca aaa cgc gta cgt tct cgt agc gac tac tac      192
   Ile Thr Tyr Trp Ala Ser Lys Arg Val Arg Ser Arg Ser Asp Tyr Tyr
       50                  55                  60
   acc gca ggc ggc aat atc act ggc ttc cag aac ggg ctg gcg att gcc      240
   Thr Ala Gly Gly Asn Ile Thr Gly Phe Gln Asn Gly Leu Ala Ile Ala
   65                  70                  75                  80
   ggg gac tat atg tcc gcc gcc tca ttc ttg ggg atc tcc gcg ctg gtg      288
   Gly Asp Tyr Met Ser Ala Ala Ser Phe Leu Gly Ile Ser Ala Leu Val
                   85                  90                  95
   ttt acc tcc ggc tat gac ggc tta att tac tcg ctg ggc ttc ctg gtg      336
   Phe Thr Ser Gly Tyr Asp Gly Leu Ile Tyr Ser Leu Gly Phe Leu Val
               100                 105                 110
   ggc tgg ccg atc att ttg ttc ctg att gcc gaa cgt ctg cgt aac ctg      384
   Gly Trp Pro Ile Ile Leu Phe Leu Ile Ala Glu Arg Leu Arg Asn Leu
           115                 120                 125
   ggg cgc tac acc ttt gcc gat gtg gcc tct tac cgt ctg aaa caa ggg      432
   Gly Arg Tyr Thr Phe Ala Asp Val Ala Ser Tyr Arg Leu Lys Gln Gly
       130                 135                 140
   ccg att cgt att ctt tcg gcc tgt ggt tct ctg gtg gtg gtg gcg ctt      480
   Pro Ile Arg Ile Leu Ser Ala Cys Gly Ser Leu Val Val Val Ala Leu
   145                 150                 155                 160
   tac ctt atc gcc cag atg gtg ggc gca ggt aaa ctg atc gag ctg ctg      528
   Tyr Leu Ile Ala Gln Met Val Gly Ala Gly Lys Leu Ile Glu Leu Leu
                   165                 170                 175
   ttt ggc ctt aac tat cac att gcg gtg gtg ctg gtc ggc gtg ctg atg      576
   Phe Gly Leu Asn Tyr His Ile Ala Val Val Leu Val Gly Val Leu Met
               180                 185                 190
   atg atg tac gtc ctg ttc ggc ggc atg ctg gcg acc acc tgg gtg caa      624
   Met Met Tyr Val Leu Phe Gly Gly Met Leu Ala Thr Thr Trp Val Gln
           195                 200                 205
   att atc aaa gcc gtg ctg ttg ctg ttc ggt gcc agc ttt atg gcc ttt      672
   Ile Ile Lys Ala Val Leu Leu Leu Phe Gly Ala Ser Phe Met Ala Phe
       210                 215                 220
   atg gtg atg aaa cac gtc ggc ttt agc ttc aac aat ctg ttc agt gaa      720
   Met Val Met Lys His Val Gly Phe Ser Phe Asn Asn Leu Phe Ser Glu
   225                 230                 235                 240
   gcg atg gcg gta cac ccg aaa ggt gtc gac atc atg aag ccg ggc ggg      768
   Ala Met Ala Val His Pro Lys Gly Val Asp Ile Met Lys Pro Gly Gly
                   245                 250                 255
   ctg gtg aaa gat ccg atc tcc gcg ctc tct ctg ggt ctg gga ctg atg      816
   Leu Val Lys Asp Pro Ile Ser Ala Leu Ser Leu Gly Leu Gly Leu Met
               260                 265                 270
   ttt ggt acg gcg ggc ttg ccg cac att ctg atg cgc ttc ttt aca gtc      864
   Phe Gly Thr Ala Gly Leu Pro His Ile Leu Met Arg Phe Phe Thr Val
           275                 280                 285
   agc gat gcc cgc gaa gca cgt aag agc gtg ttc tac gcc acc ggg ttt      912
   Ser Asp Ala Arg Glu Ala Arg Lys Ser Val Phe Tyr Ala Thr Gly Phe
       290                 295                 300
   atg ggc tac ttc tat att ctg acc ttt att atc ggc ttc ggc gcg atc      960
   Met Gly Tyr Phe Tyr Ile Leu Thr Phe Ile Ile Gly Phe Gly Ala Ile
   305                 310                 315                 320
   atg ctg gtt ggt gcg aat ccg gaa tat aaa gac gcg gcg ggc cat ctg     1008
   Met Leu Val Gly Ala Asn Pro Glu Tyr Lys Asp Ala Ala Gly His Leu
                   325                 330                 335
   att ggt ggt aac aac atg gcg gcc gtt cac ctg gcg aat gca gtg ggc     1056
   Ile Gly Gly Asn Asn Met Ala Ala Val His Leu Ala Asn Ala Val Gly
               340                 345                 350
   ggc aac ctg ttc ctc ggt ttt att tca gcg gtt gct ttc gcc act atc     1104
   Gly Asn Leu Phe Leu Gly Phe Ile Ser Ala Val Ala Phe Ala Thr Ile
           355                 360                 365
   ctc gcg gtg gtt gcg ggt ctg acg ctg gcg ggc gca tcc gcg gtt tcg     1152
   Leu Ala Val Val Ala Gly Leu Thr Leu Ala Gly Ala Ser Ala Val Ser
       370                 375                 380
   cat gac ttg tac gct aac gtc ttc aaa aaa ggc gcg acc gaa cgt gaa     1200
   His Asp Leu Tyr Ala Asn Val Phe Lys Lys Gly Ala Thr Glu Arg Glu
   385                 390                 395                 400
   gag ctg cgg gta tca aaa atc acc gta ctg atc ctc ggc gtg att gcg     1248
   Glu Leu Arg Val Ser Lys Ile Thr Val Leu Ile Leu Gly Val Ile Ala
                   405                 410                 415
   att atc ctc ggc gtg ctg ttt gag aat cag aac atc gcc ttt atg gtg     1296
   Ile Ile Leu Gly Val Leu Phe Glu Asn Gln Asn Ile Ala Phe Met Val
               420                 425                 430
   ggg ctg gcg ttt gcc atc gcg gcg agc tgt aac ttc ccg atc att ctg      1344
   Gly Leu Ala Phe Ala Ile Ala Ala Ser Cys Asn Phe Pro Ile Ile Leu
           435                     440                     445
   ctt tct atg tac tgg tcg aaa ctg acc acg cgt ggc gcg atg atg ggt      1392
   Leu Ser Met Tyr Trp Ser Lys Leu Thr Thr Arg Gly Ala Met Met Gly
       450                     455                     460
   ggc tgg ctg ggg ctg att acc gca gta gta ctg atg atc ctc ggc ccg      1440
   Gly Trp Leu Gly Leu Ile Thr Ala Val Val Leu Met Ile Leu Gly Pro
   465                     470                     475                     480
   acg att tgg gta cag atc ctt ggt cac gaa aaa gcc atc ttc ccg tat      1488
   Thr Ile Trp Val Gln Ile Leu Gly His Glu Lys Ala Ile Phe Pro Tyr
                   485                     490                     495
   gaa tac ccg gcg ctg ttc tct atc acc gtg gca ttc ctc ggc atc tgg      1536
   Glu Tyr Pro Ala Leu Phe Ser Ile Thr Val Ala Phe Leu Gly Ile Trp
               500                     505                     510
   ttc ttc tcg gca acc gat aac tca gcg gaa ggc gcg cgt gag cgt gaa      1584
   Phe Phe Ser Ala Thr Asp Asn Ser Ala Glu Gly Ala Arg Glu Arg Glu
           515                     520                     525
   ctg ttc cgc gcg cag ttt atc cgc tcc cag acc ggc ttt ggc gtt gag      1632
   Leu Phe Arg Ala Gln Phe Ile Arg Ser Gln Thr Gly Phe Gly Val Glu
       530                     535                     540
   caa ggc cgc gcg cat taa                                              1650
   Gln Gly Arg Ala His
   545
<210> 2
   <211> 549
   <212> PRT
   <213> Escherichia coli
<400> 2
   Met Lys Arg Val Leu Thr Ala Leu Ala Ala Thr Leu Pro Phe Ala Ala
   1               5                   10                  15
   Asn Ala Ala Asp Ala Ile Ser Gly Ala Val Glu Arg Gln Pro Thr Asn
               20                  25                  30
   Trp Gln Ala Ile Ile Met Phe Leu Ile Phe Val Val Phe Thr Leu Gly
           35                  40                  45
   Ile Thr Tyr Trp Ala Ser Lys Arg Val Arg Ser Arg Ser Asp Tyr Tyr
       50                  55                  60
   Thr Ala Gly Gly Asn Ile Thr Gly Phe Gln Asn Gly Leu Ala Ile Ala
   65                  70                  75                  80
   Gly Asp Tyr Met Ser Ala Ala Ser Phe Leu Gly Ile Ser Ala Leu Val
                   85                  90                  95
   Phe Thr Ser Gly Tyr Asp Gly Leu Ile Tyr Ser Leu Gly Phe Leu Val
   100 105 110
   Gly Trp Pro Ile Ile Leu Phe Leu Ile Ala Glu Arg Leu Arg Asn Leu
   115 120 125
   Gly Arg Tyr Thr Phe Ala Asp Val Ala Ser Tyr Arg Leu Lys Gln Gly
   130 135 140
   Pro Ile Arg Ile Leu Ser Ala Cys Gly Ser Leu Val Val Val Ala Leu
   145 150 155 160
   Tyr Leu Ile Ala Gln Met Val Gly Ala Gly Lys Leu Ile Glu Leu Leu
   165 170 175
   Phe Gly Leu Asn Tyr His Ile Ala Val Val Leu Val Gly Val Leu Met
   180 185 190
   Met Met Tyr Val Leu Phe Gly Gly Met Leu Ala Thr Thr Trp Val Gln
   195 200 205
   Ile Ile Lys Ala Val Leu Leu Leu Phe Gly Ala Ser Phe Met Ala Phe
   210 215 220
   Met Val Met Lys His Val Gly Phe Ser Phe Asn Asn Leu Phe Ser Glu
   225 230 235 240
   Ala Met Ala Val His Pro Lys Gly Val Asp Ile Met Lys Pro Gly Gly
   245 250 255
   Leu Val Lys Asp Pro Ile Ser Ala Leu Ser Leu Gly Leu Gly Leu Met
   260 265 270
   Phe Gly Thr Ala Gly Leu Pro His Ile Leu Met Arg Phe Phe Thr Val
   275 280 285
   Ser Asp Ala Arg Glu Ala Arg Lys Ser Val Phe Tyr Ala Thr Gly Phe
   290 295 300
   Met Gly Tyr Phe Tyr Ile Leu Thr Phe Ile Ile Gly Phe Gly Ala Ile
   305 310 315 320
   Met Leu Val Gly Ala Asn Pro Glu Tyr Lys Asp Ala Ala Gly His Leu
   325 330 335
   Ile Gly Gly Asn Asn Met Ala Ala Val His Leu Ala Asn Ala Val Gly
   340 345 350
   Gly Asn Leu Phe Leu Gly Phe Ile Ser Ala Val Ala Phe Ala Thr Ile
   355 360 365
   Leu Ala Val Val Ala Gly Leu Thr Leu Ala Gly Ala Ser Ala Val Ser
       370                 375                 380
   His Asp Leu Tyr Ala Asn Val Phe Lys Lys Gly Ala Thr Glu Arg Glu
   385                 390                 395                 400
   Glu Leu Arg Val Ser Lys Ile Thr Val Leu Ile Leu Gly Val Ile Ala
                   405                 410                 415
   Ile Ile Leu Gly Val Leu Phe Glu Asn Gln Asn Ile Ala Phe Met Val
               420                 425                 430
   Gly Leu Ala Phe Ala Ile Ala Ala Ser Cys Asn Phe Pro Ile Ile Leu
           435                 440                 445
   Leu Ser Met Tyr Trp Ser Lys Leu Thr Thr Arg Gly Ala Met Met Gly
       450                 455                 460
   Gly Trp Leu Gly Leu Ile Thr Ala Val Val Leu Met Ile Leu Gly Pro
   465                 470                 475                 480
   Thr Ile Trp Val Gln Ile Leu Gly His Glu Lys Ala Ile Phe Pro Tyr
                   485                 490                 495
   Glu Tyr Pro Ala Leu Phe Ser Ile Thr Val Ala Phe Leu Gly Ile Trp
               500                 505                 510
   Phe Phe Ser Ala Thr Asp Asn Ser Ala Glu Gly Ala Arg Glu Arg Glu
           515                 520                 525
   Leu Phe Arg Ala Gln Phe Ile Arg Ser Gln Thr Gly Phe Gly Val Glu
       530                 535                 540
   Gln Gly Arg Ala His
   545
<210> 3
   <211> 1650
   <212> DNA
   <213> Salmonella typhimurium
<220>
   <221> CDS
   <222> (1)..(1650)
   <223> yjcG Kodierregion
<400> 3
   atg aag aga gtt ctg acg gcg ctt gcc gcc gca ctc ccc ttc gcc gct         48
   Met Lys Arg Val Leu Thr Ala Leu Ala Ala Ala Leu Pro Phe Ala Ala
   1               5                   10                  15
   cat gcg gcg gat gcc att agc ggc gcg gtt gaa cgc cag ccc acc aac         96
   His Ala Ala Asp Ala Ile Ser Gly Ala Val Glu Arg Gln Pro Thr Asn
               20                  25                  30
   tgg cag gcg att atc atg ttt ttg att ttc gtc gtg ttt acg ctc ggt      144
   Trp Gln Ala Ile Ile Met Phe Leu Ile Phe Val Val Phe Thr Leu Gly
           35                  40                  45
   att acc tac tgg gcc tct aaa cgc gta cgt tcc cgt agc gac tac tac      192
   Ile Thr Tyr Trp Ala Ser Lys Arg Val Arg Ser Arg Ser Asp Tyr Tyr
       50                  55                  60
   acc gct ggc ggc aat atc acc ggg ttc cag aac ggc ctg gcg att gcc      240
   Thr Ala Gly Gly Asn Ile Thr Gly Phe Gln Asn Gly Leu Ala Ile Ala
   65                  70                  75                  80
   ggc gac tat atg tct gcc gcg tca ttt ctc ggc att tcc gcg ctg gtg      288
   Gly Asp Tyr Met Ser Ala Ala Ser Phe Leu Gly Ile Ser Ala Leu Val
                   85                  90                  95
   ttt acc tcc ggt tat gac ggg ctg atc tat tcg ctg ggc ttc ctt gtc      336
   Phe Thr Ser Gly Tyr Asp Gly Leu Ile Tyr Ser Leu Gly Phe Leu Val
               100                 105                 110
   ggc tgg cca atc atc ctg ttt ttg att gcc gag cgc ctg cgt aat ctg      384
   Gly Trp Pro Ile Ile Leu Phe Leu Ile Ala Glu Arg Leu Arg Asn Leu
           115                 120                 125
   gga cgt tat act ttt gct gac gtt gcc tct tat cgc ctg aaa cag ggg      432
   Gly Arg Tyr Thr Phe Ala Asp Val Ala Ser Tyr Arg Leu Lys Gln Gly
       130                 135                 140
   cca atc cgt att ctt tcg gcc tgt ggc tcc ctg gta gtg gtg gcg ctt      480
   Pro Ile Arg Ile Leu Ser Ala Cys Gly Ser Leu Val Val Val Ala Leu
   145                 150                 155                 160
   tat ctc atc gcc caa atg gtc ggc gcc ggt aaa ctg att gaa ctg ctg      528
   Tyr Leu Ile Ala Gln Met Val Gly Ala Gly Lys Leu Ile Glu Leu Leu
                   165                 170                 175
   ttc ggc ctc aac tat cac atc gct gtg gtg ctg gtc ggc gtg ctg atg      576
   Phe Gly Leu Asn Tyr His Ile Ala Val Val Leu Val Gly Val Leu Met
               180                 185                 190
   atg atg tac gtg ctg ttc ggc ggg atg ctg gcg aca acg tgg gtg caa      624
   Met Met Tyr Val Leu Phe Gly Gly Met Leu Ala Thr Thr Trp Val Gln
           195                 200                 205
   att atc aaa gcc gtc ctg ttg ctg ttt ggc gcc agt ttt atg gcc ttt      672
   Ile Ile Lys Ala Val Leu Leu Leu Phe Gly Ala Ser Phe Met Ala Phe
       210                 215                 220
   atg gtg atg aaa cac gtc ggc ttt agc ttc aat aat ctg ttt acc gaa      720
   Met Val Met Lys His Val Gly Phe Ser Phe Asn Asn Leu Phe Thr Glu
   225                 230                 235                 240
   gcc atg gcg gta cac ccg aaa ggc acg gcg att atg agc cca gga gga      768
   Ala Met Ala Val His Pro Lys Gly Thr Ala Ile Met Ser Pro Gly Gly
                   245                 250                 255
   ttg gtg caa gat ccg att tcg gca ttg tcg ttg ggt ctg gga ctg atg      816
   Leu Val Gln Asp Pro Ile Ser Ala Leu Ser Leu Gly Leu Gly Leu Met
               260                 265                 270
   ttc ggc acc gcc ggc ttg ccg cat att ctg atg cgt ttc ttt acg gtc      864
   Phe Gly Thr Ala Gly Leu Pro His Ile Leu Met Arg Phe Phe Thr Val
           275                 280                 285
   agc gat gcc cgc gaa gcg cgc aag agc gtg ttc tac gcc acc ggt ttt      912
   Ser Asp Ala Arg Glu Ala Arg Lys Ser Val Phe Tyr Ala Thr Gly Phe
       290                 295                 300
   atg ggc tat ttc tac att ctg acc ttt att atc ggc ttc ggc gct atc      960
   Met Gly Tyr Phe Tyr Ile Leu Thr Phe Ile Ile Gly Phe Gly Ala Ile
   305                 310                 315                 320
   atg ctg gtg ggg gcg aat ccc gcc tat aaa gat gcc gca ggc gcg ctg     1008
   Met Leu Val Gly Ala Asn Pro Ala Tyr Lys Asp Ala Ala Gly Ala Leu
                   325                 330                 335
   att ggc ggc aat aac atg gcg gcg gtg cat ctg gcc aac gcg gta ggc     1056
   Ile Gly Gly Asn Asn Met Ala Ala Val His Leu Ala Asn Ala Val Gly
               340                 345                 350
   ggc aac ctg ttc ctc ggc ttt att tcg gca gtg gcg ttt gcc acc att     1104
   Gly Asn Leu Phe Leu Gly Phe Ile Ser Ala Val Ala Phe Ala Thr Ile
           355                 360                 365
   ctg gcg gtg gtc gca ggt ctg acg ctg gcg ggc gca tcg gcg gtg tcg     1152
   Leu Ala Val Val Ala Gly Leu Thr Leu Ala Gly Ala Ser Ala Val Ser
       370                 375                 380
   cat gac ttg tac gcc aac gtg ttc cgc aaa ggc gca acc gaa cgt gaa     1200
   His Asp Leu Tyr Ala Asn Val Phe Arg Lys Gly Ala Thr Glu Arg Glu
   385                 390                 395                 400
   gag ctg aag gtg tcg aaa atc acc gtc ctg gtg ctg ggc gtg atc gcc     1248
   Glu Leu Lys Val Ser Lys Ile Thr Val Leu Val Leu Gly Val Ile Ala
                   405                 410                 415
   att atc ctc ggc gtc ctg ttt gaa aat cag aac atc gcc ttt atg gtg     1296
   Ile Ile Leu Gly Val Leu Phe Glu Asn Gln Asn Ile Ala Phe Met Val
               420                 425                 430
   ggc ctg gca ttt gct atc gcc gcg agc tgc aac ttc ccc atc att ctg     1344
   Gly Leu Ala Phe Ala Ile Ala Ala Ser Cys Asn Phe Pro Ile Ile Leu
           435                 440                 445
   ctt tcc atg tac tgg tca aaa ctg acc acg cgc ggc gct atg ctg ggc     1392
   Leu Ser Met Tyr Trp Ser Lys Leu Thr Thr Arg Gly Ala Met Leu Gly
       450                 455                 460
   ggc tgg tta ggt tta ctg aca gcg gtg gtg ctg atg att ctt ggc cct     1440
   Gly Trp Leu Gly Leu Leu Thr Ala Val Val Leu Met Ile Leu Gly Pro
   465                 470                 475                 480
   acc att tgg gtg cag atc ctc ggc cac gaa aaa gcg atc ttc ccg tat     1488
   Thr Ile Trp Val Gln Ile Leu Gly His Glu Lys Ala Ile Phe Pro Tyr
                   485                 490                 495
   gag tat ccg gcg ctg ttc tct atc agc gtg gcg ttc ctg ggg atc tgg     1536
   Glu Tyr Pro Ala Leu Phe Ser Ile Ser Val Ala Phe Leu Gly Ile Trp
               500                 505                 510
   ttc ttc tcg gcc acc gat aac tcg gca gaa ggc aac cgt gaa cgt gag     1584
   Phe Phe Ser Ala Thr Asp Asn Ser Ala Glu Gly Asn Arg Glu Arg Glu
           515                 520                 525
   cag ttc cgc gct cag ttt atc cgc tcc caa acg gga ttc ggc gta caa     1632
   Gln Phe Arg Ala Gln Phe Ile Arg Ser Gln Thr Gly Phe Gly Val Gln
       530                 535                 540
   caa ggg cgt gcg cat taa                                             1650
   Gln Gly Arg Ala His
   545
<210> 4
   <211> 549
   <212> PRT
   <213> Salmonella typhimurium
<400> 4
   Met Lys Arg Val Leu Thr Ala Leu Ala Ala Ala Leu Pro Phe Ala Ala
   1               5                   10                  15
   His Ala Ala Asp Ala Ile Ser Gly Ala Val Glu Arg Gln Pro Thr Asn
               20                  25                  30
   Trp Gln Ala Ile Ile Met Phe Leu Ile Phe Val Val Phe Thr Leu Gly
           35                  40                  45
   Ile Thr Tyr Trp Ala Ser Lys Arg Val Arg Ser Arg Ser Asp Tyr Tyr
       50                  55                  60
   Thr Ala Gly Gly Asn Ile Thr Gly Phe Gln Asn Gly Leu Ala Ile Ala
   65                  70                  75                  80
   Gly Asp Tyr Met Ser Ala Ala Ser Phe Leu Gly Ile Ser Ala Leu Val
                   85                  90                  95
   Phe Thr Ser Gly Tyr Asp Gly Leu Ile Tyr Ser Leu Gly Phe Leu Val
               100                 105                 110
   Gly Trp Pro Ile Ile Leu Phe Leu Ile Ala Glu Arg Leu Arg Asn Leu
           115                 120                 125
   Gly Arg Tyr Thr Phe Ala Asp Val Ala Ser Tyr Arg Leu Lys Gln Gly
       130                 135                 140
   Pro Ile Arg Ile Leu Ser Ala Cys Gly Ser Leu Val Val Val Ala Leu
   145                 150                 155                 160
   Tyr Leu Ile Ala Gln Met Val Gly Ala Gly Lys Leu Ile Glu Leu Leu
                   165                 170                 175
   Phe Gly Leu Asn Tyr His Ile Ala Val Val Leu Val Gly Val Leu Met
               180                 185                 190
   Met Met Tyr Val Leu Phe Gly Gly Met Leu Ala Thr Thr Trp Val Gln
           195                 200                 205
   Ile Ile Lys Ala Val Leu Leu Leu Phe Gly Ala Ser Phe Met Ala Phe
       210                 215                 220
   Met Val Met Lys His Val Gly Phe Ser Phe Asn Asn Leu Phe Thr Glu
   225                 230                 235                 240
   Ala Met Ala Val His Pro Lys Gly Thr Ala Ile Met Ser Pro Gly Gly
                   245                 250                 255
   Leu Val Gln Asp Pro Ile Ser Ala Leu Ser Leu Gly Leu Gly Leu Met
               260                 265                 270
   Phe Gly Thr Ala Gly Leu Pro His Ile Leu Met Arg Phe Phe Thr Val
           275                 280                 285
   Ser Asp Ala Arg Glu Ala Arg Lys Ser Val Phe Tyr Ala Thr Gly Phe
       290                 295                 300
   Met Gly Tyr Phe Tyr Ile Leu Thr Phe Ile Ile Gly Phe Gly Ala Ile
   305                 310                 315                 320
   Met Leu Val Gly Ala Asn Pro Ala Tyr Lys Asp Ala Ala Gly Ala Leu
                   325                 330                 335
   Ile Gly Gly Asn Asn Met Ala Ala Val His Leu Ala Asn Ala Val Gly
               340                 345                 350
   Gly Asn Leu Phe Leu Gly Phe Ile Ser Ala Val Ala Phe Ala Thr Ile
           355                 360                 365
   Leu Ala Val Val Ala Gly Leu Thr Leu Ala Gly Ala Ser Ala Val Ser
       370                 375                 380
   His Asp Leu Tyr Ala Asn Val Phe Arg Lys Gly Ala Thr Glu Arg Glu
   385                 390                 395                 400
   Glu Leu Lys Val Ser Lys Ile Thr Val Leu Val Leu Gly Val Ile Ala
                   405                 410                 415
   Ile Ile Leu Gly Val Leu Phe Glu Asn Gln Asn Ile Ala Phe Met Val
               420                 425                 430
   Gly Leu Ala Phe Ala Ile Ala Ala Ser Cys Asn Phe Pro Ile Ile Leu
           435                 440                 445
   Leu Ser Met Tyr Trp Ser Lys Leu Thr Thr Arg Gly Ala Met Leu Gly
       450                 455                 460
   Gly Trp Leu Gly Leu Leu Thr Ala Val Val Leu Met Ile Leu Gly Pro
   465                 470                 475                 480
   Thr Ile Trp Val Gln Ile Leu Gly His Glu Lys Ala Ile Phe Pro Tyr
                   485                 490                 495
   Glu Tyr Pro Ala Leu Phe Ser Ile Ser Val Ala Phe Leu Gly Ile Trp
               500                 505                 510
   Phe Phe Ser Ala Thr Asp Asn Ser Ala Glu Gly Asn Arg Glu Arg Glu
           515                 520                 525
   Gln Phe Arg Ala Gln Phe Ile Arg Ser Gln Thr Gly Phe Gly Val Gln
       530                 535                 540
   Gln Gly Arg Ala His
   545
<210> 5
   <211> 1650
   <212> DNA
   <213> Shigella flexneri
<220>
   <221> CDS
   <222> (1)..(1650)
   <223> yjcG Kodierregion
<400> 5
   atg aaa aga gtt ctg acg gcg ctt gcc gcc aca ctc cct ttc gca gct 48
   Met Lys Arg Val Leu Thr Ala Leu Ala Ala Thr Leu Pro Phe Ala Ala
   1 5 10 15
   aac gcc gcg gat gct att agc ggg gcc gta gag cgc cag cca acg aac 96
   Asn Ala Ala Asp Ala Ile Ser Gly Ala Val Glu Arg Gln Pro Thr Asn
   20 25 30
   tgg cag gcg att att atg ttc ctg att ttc gtc gtg ttt acg ctc ggc 144
   Trp Gln Ala Ile Ile Met Phe Leu Ile Phe Val Val Phe Thr Leu Gly
   35 40 45
   att acc tac tgg gca tca aaa cgc gta cgt tct cgt agc gac tac tac 192
   Ile Thr Tyr Trp Ala Ser Lys Arg Val Arg Ser Arg Ser Asp Tyr Tyr
   50 55 60
   acc gca ggc ggc aat atc act ggc ttc cag aac ggg ctg gcg att gcc 240
   Thr Ala Gly Gly Asn Ile Thr Gly Phe Gln Asn Gly Leu Ala Ile Ala
   65 70 75 80
   ggg gac tat atg tcc gcc gcc tca ttc ttg ggg atc tcc gcg ctg gtg 288
   Gly Asp Tyr Met Ser Ala Ala Ser Phe Leu Gly Ile Ser Ala Leu Val
   85 90 95
   ttt acc tcc ggc tat gac ggg ctg atc tac tcg ttg ggc ttc ctg gtg 336
   Phe Thr Ser Gly Tyr Asp Gly Leu Ile Tyr Ser Leu Gly Phe Leu Val
   100 105 110
   ggc tgg cca atc att ctg ttt ctg att gcc gaa cgt ctg cgt aac ctg 384
   Gly Trp Pro Ile Ile Leu Phe Leu Ile Ala Glu Arg Leu Arg Asn Leu
   115 120 125
   gga cgc tac acc tct gcc gat gtg gcc tct tat cgt ctg aaa caa ggg 432
   Gly Arg Tyr Thr Ser Ala Asp Val Ala Ser Tyr Arg Leu Lys Gln Gly
   130 135 140
   ccg att cgt att ctt tcg gcc tgt ggt tct ctg gta gtg gtg gcg ctt      480
   Pro Ile Arg Ile Leu Ser Ala Cys Gly Ser Leu Val Val Val Ala Leu
   145                 150                 155                 160
   tac ctt atc gct cag atg gta ggc gca ggt aaa ctg atc gag ctg ctg      528
   Tyr Leu Ile Ala Gln Met Val Gly Ala Gly Lys Leu Ile Glu Leu Leu
                   165                 170                 175
   ttt ggc ctt aac tat cac att gcg gtg gtg ctg gtc ggc gtg ctg atg      576
   Phe Gly Leu Asn Tyr His Ile Ala Val Val Leu Val Gly Val Leu Met
               180                 185                 190
   atg atg tac gtc ctg ttc ggc ggc atg ctg gcg acc acc tgg gta caa      624
   Met Met Tyr Val Leu Phe Gly Gly Met Leu Ala Thr Thr Trp Val Gln
           195                 200                 205
   att atc aaa gct gtg ctg ttg ctg ttc ggt gcc agc ttt atg gcc ttt      672
   Ile Ile Lys Ala Val Leu Leu Leu Phe Gly Ala Ser Phe Met Ala Phe
       210                 215                 220
   atg gtg atg aaa cac gtc ggc ttt agc ttc aac aat ctg ttc agc gaa      720
   Met Val Met Lys His Val Gly Phe Ser Phe Asn Asn Leu Phe Ser Glu
   225                 230                 235                 240
   gcg atg gcg gta cac ccg aaa ggt gtc gac atc atg aaa ccg ggc gga      768
   Ala Met Ala Val His Pro Lys Gly Val Asp Ile Met Lys Pro Gly Gly
                   245                 250                 255
   ctg gtg aaa gat ccg atc tcc gcg ctc tct ctg ggg ctg gga ctg atg      816
   Leu Val Lys Asp Pro Ile Ser Ala Leu Ser Leu Gly Leu Gly Leu Met
               260                 265                 270
   ttt ggt acg gcg ggc ttg ccg cac att ctg atg cgc ttc ttt aca gtc      864
   Phe Gly Thr Ala Gly Leu Pro His Ile Leu Met Arg Phe Phe Thr Val
           275                 280                 285
   agc gat gcc cgc gaa gca cgt aag agc gtg ttc tac gcc acc gga ttt      912
   Ser Asp Ala Arg Glu Ala Arg Lys Ser Val Phe Tyr Ala Thr Gly Phe
       290                 295                 300
   atg ggc tac ttc tat att ctg acc ttt att atc ggc ttc ggc gcg atc      960
   Met Gly Tyr Phe Tyr Ile Leu Thr Phe Ile Ile Gly Phe Gly Ala Ile
   305                 310                 315                 320
   atg ctt gtt ggt gcg aat ccg gaa tat aaa gac gcg gcg ggc cat ctg     1008
   Met Leu Val Gly Ala Asn Pro Glu Tyr Lys Asp Ala Ala Gly His Leu
                   325                 330                 335
   att ggt ggt aac aac atg gcg gcc gtt cac ctg gcg aat gca gtg ggc     1056
   Ile Gly Gly Asn Asn Met Ala Ala Val His Leu Ala Asn Ala Val Gly
               340                 345                 350
   ggc aac cta ttc ctc ggt ttt att tca gcg gtt gct ttc gcc act atc     1104
   Gly Asn Leu Phe Leu Gly Phe Ile Ser Ala Val Ala Phe Ala Thr Ile
           355                 360                 365
   ctc gcg gtg gtt gcg gat ctg acg ctg gcg ggc gca tcg gcg gtt tcg     1152
   Leu Ala Val Val Ala Asp Leu Thr Leu Ala Gly Ala Ser Ala Val Ser
       370                 375                 380
   cat gac ttg tac gct aac gtc ttc aaa aaa ggc gcg acc gaa cgt gaa     1200
   His Asp Leu Tyr Ala Asn Val Phe Lys Lys Gly Ala Thr Glu Arg Glu
   385                 390                 395                 400
   gag ctg cgg gta tca aaa atc acc gta ctg atc ctc ggc gtg att gcg     1248
   Glu Leu Arg Val Ser Lys Ile Thr Val Leu Ile Leu Gly Val Ile Ala
                   405                 410                 415
   att atc ctc ggc gtg ctg ttt gag aat cag aac atc gcc ttt atg gta      1296
   Ile Ile Leu Gly Val Leu Phe Glu Asn Gln Asn Ile Ala Phe Met Val
               420                 425                 430
   ggc ctt gcg ttt gcc atc gcg gcg agc tgt aac ttc ccg atc att ctg      1344
   Gly Leu Ala Phe Ala Ile Ala Ala Ser Cys Asn Phe Pro Ile Ile Leu
           435                 440                 445
   ctt tcc atg tac tgg tcg aaa ctg act acc cgt ggc gcg atg ctg ggc      1392
   Leu Ser Met Tyr Trp Ser Lys Leu Thr Thr Arg Gly Ala Met Leu Gly
       450                 455                 460
   ggc tgg ctg ggg ctg att acc gca gtg gtg ctg atg atc ctc ggc ccg      1440
   Gly Trp Leu Gly Leu Ile Thr Ala Val Val Leu Met Ile Leu Gly Pro
   465                 470                 475                 480
   act att tgg gta cag atc ctc ggt cac gaa aaa gcc atc ttc ccg tat      1488
   Thr Ile Trp Val Gln Ile Leu Gly His Glu Lys Ala Ile Phe Pro Tyr
                   485                 490                 495
   gaa tac ccg gcg ctg ttc tct atc agc gtg gca ttc ctc ggc atc tgg      1536
   Glu Tyr Pro Ala Leu Phe Ser Ile Ser Val Ala Phe Leu Gly Ile Trp
               500                 505                 510
   ttc ttc tcg gca acc gat aac tca gcg gaa ggg gcg cgc gag cgt gaa      1584
   Phe Phe Ser Ala Thr Asp Asn Ser Ala Glu Gly Ala Arg Glu Arg Glu
           515                 520                 525
   ctg ttc cgc gcg cag ttt atc cgc tcc cag acc ggc ttt ggc gtt gag      1632
   Leu Phe Arg Ala Gln Phe Ile Arg Ser Gln Thr Gly Phe Gly Val Glu
       530                 535                 540
   caa ggc cgc gca cat taa                                              1650
   Gln Gly Arg Ala His
   545
<210> 6
   <211> 549
   <212> PRT
   <213> Shigella flexneri
<400> 6
   Met Lys Arg Val Leu Thr Ala Leu Ala Ala Thr Leu Pro Phe Ala Ala
   1               5                   10                  15
   Asn Ala Ala Asp Ala Ile Ser Gly Ala Val Glu Arg Gln Pro Thr Asn
               20                  25                  30
   Trp Gln Ala Ile Ile Met Phe Leu Ile Phe Val Val Phe Thr Leu Gly
           35                  40                  45
   Ile Thr Tyr Trp Ala Ser Lys Arg Val Arg Ser Arg Ser Asp Tyr Tyr
       50                  55                  60
   Thr Ala Gly Gly Asn Ile Thr Gly Phe Gln Asn Gly Leu Ala Ile Ala
   65                  70                  75                  80
   Gly Asp Tyr Met Ser Ala Ala Ser Phe Leu Gly Ile Ser Ala Leu Val
                   85                  90                  95
   Phe Thr Ser Gly Tyr Asp Gly Leu Ile Tyr Ser Leu Gly Phe Leu Val
               100                 105                 110
   Gly Trp Pro Ile Ile Leu Phe Leu Ile Ala Glu Arg Leu Arg Asn Leu
           115                 120                 125
   Gly Arg Tyr Thr Ser Ala Asp Val Ala Ser Tyr Arg Leu Lys Gln Gly
       130                 135                 140
   Pro Ile Arg Ile Leu Ser Ala Cys Gly Ser Leu Val Val Val Ala Leu
   145                 150                 155                 160
   Tyr Leu Ile Ala Gln Met Val Gly Ala Gly Lys Leu Ile Glu Leu Leu
                   165                 170                 175
   Phe Gly Leu Asn Tyr His Ile Ala Val Val Leu Val Gly Val Leu Met
               180                 185                 190
   Met Met Tyr Val Leu Phe Gly Gly Met Leu Ala Thr Thr Trp Val Gln
           195                 200                 205
   Ile Ile Lys Ala Val Leu Leu Leu Phe Gly Ala Ser Phe Met Ala Phe
       210                 215                 220
   Met Val Met Lys His Val Gly Phe Ser Phe Asn Asn Leu Phe Ser Glu
   225                 230                 235                 240
   Ala Met Ala Val His Pro Lys Gly Val Asp Ile Met Lys Pro Gly Gly
                   245                 250                 255
   Leu Val Lys Asp Pro Ile Ser Ala Leu Ser Leu Gly Leu Gly Leu Met
               260                 265                 270
   Phe Gly Thr Ala Gly Leu Pro His Ile Leu Met Arg Phe Phe Thr Val
           275                 280                 285
   Ser Asp Ala Arg Glu Ala Arg Lys Ser Val Phe Tyr Ala Thr Gly Phe
       290                 295                 300
   Met Gly Tyr Phe Tyr Ile Leu Thr Phe Ile Ile Gly Phe Gly Ala Ile
   305                 310                 315                 320
   Met Leu Val Gly Ala Asn Pro Glu Tyr Lys Asp Ala Ala Gly His Leu
                   325                 330                 335
   Ile Gly Gly Asn Asn Met Ala Ala Val His Leu Ala Asn Ala Val Gly
               340                 345                 350
   Gly Asn Leu Phe Leu Gly Phe Ile Ser Ala Val Ala Phe Ala Thr Ile
           355                 360                 365
   Leu Ala Val Val Ala Asp Leu Thr Leu Ala Gly Ala Ser Ala Val Ser
       370                 375                 380
   His Asp Leu Tyr Ala Asn Val Phe Lys Lys Gly Ala Thr Glu Arg Glu
   385                 390                 395                 400
   Glu Leu Arg Val Ser Lys Ile Thr Val Leu Ile Leu Gly Val Ile Ala
                   405                 410                 415
   Ile Ile Leu Gly Val Leu Phe Glu Asn Gln Asn Ile Ala Phe Met Val
               420                 425                 430
   Gly Leu Ala Phe Ala Ile Ala Ala Ser Cys Asn Phe Pro Ile Ile Leu
           435                 440                 445
   Leu Ser Met Tyr Trp Ser Lys Leu Thr Thr Arg Gly Ala Met Leu Gly
       450                 455                 460
   Gly Trp Leu Gly Leu Ile Thr Ala Val Val Leu Met Ile Leu Gly Pro
   465                 470                 475                 480
   Thr Ile Trp Val Gln Ile Leu Gly His Glu Lys Ala Ile Phe Pro Tyr
                   485                 490                 495
   Glu Tyr Pro Ala Leu Phe Ser Ile Ser Val Ala Phe Leu Gly Ile Trp
               500                 505                 510
   Phe Phe Ser Ala Thr Asp Asn Ser Ala Glu Gly Ala Arg Glu Arg Glu
           515                 520                 525
   Leu Phe Arg Ala Gln Phe Ile Arg Ser Gln Thr Gly Phe Gly Val Glu
       530                 535                 540
   Gln Gly Arg Ala His
   545
<210> 7
   <211> 26
   <212> DNA
   <213> Artificial Sequence
<220>
   <223> Primer yjcG-1
<220>
   <221> misc_feature
   <223> Primer yjcG-1
<400> 7
   gatcaagctt atccggccta cattcg 26
<210> 8
   <211> 31
   <212> DNA
   <213> Artificial Sequence
<220>
   <223> Primer yjcG-2
<220>
   <221> misc_feature
   <223> Primer yjcg-2
<400> 8
   gatctagagc tcgattaatg cgcgcggcct t 31
<210> 9
   <211> 2084
   <212> DNA
   <213> Corynebacterium glutamicum
<220>
   <221> CDS
   <222> (421)..(2067)
<400> 9
   gatcaagctt atccggccta cattcggcaa gggttacccg agcgttaacc ttctcccata      60
   agggagcggg aattaaaaca atccctacat tacctctgga gaatctgtga tgaatggcac     120
   tatttatcag cggatagaag acaatgcgca tttcagggag ttagtcgaaa aacggcaacg     180
   gtttgccacc atcctgtcga ttattatgct ggcagtttat atcggcttta ttttactgat     240
   cgccttcgcg cccggctggc tgggcacgcc gctgaatccg aacaccagcg tcacacgcgg     300
   tattccaatt ggtgttggag tgattgtgat ctcctttgtt ctcaccggta tctacatctg     360
   gcgggcgaac ggcgaattcg accgtcttaa taatgaagtc ctgcatgagg tacaagcatc     420
   atg aaa aga gtt ctg acg gcg ctt gcc gcc aca ctc cct ttc gca gct       468
   Met Lys Arg Val Leu Thr Ala Leu Ala Ala Thr Leu Pro Phe Ala Ala
   1               5                   10                  15
   aac gcc gcg gat gct att agc ggg gcc gta gag cgc cag cca acg aac       516
   Asn Ala Ala Asp Ala Ile Ser Gly Ala Val Glu Arg Gln Pro Thr Asn
               20                  25                  30
   tgg cag gcg att att atg ttc ctg att ttc gtc gtg ttt acg ctc ggc       564
   Trp Gln Ala Ile Ile Met Phe Leu Ile Phe Val Val Phe Thr Leu Gly
           35                  40                  45
   att acc tac tgg gca tca aaa cgc gta cgt tct cgt agc gac tac tac       612
   Ile Thr Tyr Trp Ala Ser Lys Arg Val Arg Ser Arg Ser Asp Tyr Tyr
       50                  55                  60
   acc gca ggc ggc aat atc act ggc ttc cag aac ggg ctg gcg att gcc       660
   Thr Ala Gly Gly Asn Ile Thr Gly Phe Gln Asn Gly Leu Ala Ile Ala
   65                  70                  75                  80
   ggg gac tat atg tcc gcc gcc tca ttc ttg ggg atc tcc gcg ctg gtg      708
   Gly Asp Tyr Met Ser Ala Ala Ser Phe Leu Gly Ile Ser Ala Leu Val
                   85                  90                  95
   ttt acc tcc ggc tat gac ggc tta att tac tcg ctg ggc ttc ctg gtg      756
   Phe Thr Ser Gly Tyr Asp Gly Leu Ile Tyr Ser Leu Gly Phe Leu Val
               100                 105                 110
   ggc tgg ccg atc att ttg ttc ctg att gcc gaa cgt ctg cgt aac ctg      804
   Gly Trp Pro Ile Ile Leu Phe Leu Ile Ala Glu Arg Leu Arg Asn Leu
           115                 120                 125
   ggg cgc tac acc ttt gcc gat gtg gcc tct tac cgt ctg aaa caa ggg      852
   Gly Arg Tyr Thr Phe Ala Asp Val Ala Ser Tyr Arg Leu Lys Gln Gly
       130                 135                 140
   ccg att cgt att ctt tcg gcc tgt ggt tct ctg gtg gtg gtg gcg ctt      900
   Pro Ile Arg Ile Leu Ser Ala Cys Gly Ser Leu Val Val Val Ala Leu
   145                 150                 155                 160
   tac ctt atc gcc cag atg gtg ggc gca ggt aaa ctg atc gag ctg ctg      948
   Tyr Leu Ile Ala Gln Met Val Gly Ala Gly Lys Leu Ile Glu Leu Leu
                   165                 170                 175
   ttt ggc ctt aac tat cac att gcg gtg gtg ctg gtc ggc gtg ctg atg      996
   Phe Gly Leu Asn Tyr His Ile Ala Val Val Leu Val Gly Val Leu Met
               180                 185                 190
   atg atg tac gtc ctg ttc ggc ggc atg ctg gcg acc acc tgg gtg caa     1044
   Met Met Tyr Val Leu Phe Gly Gly Met Leu Ala Thr Thr Trp Val Gln
           195                 200                 205
   att atc aaa gcc gtg ctg ttg ctg ttc ggt gcc agc ttt atg gcc ttt     1092
   Ile Ile Lys Ala Val Leu Leu Leu Phe Gly Ala Ser Phe Met Ala Phe
       210                 215                 220
   atg gtg atg aaa cac gtc ggc ttt agc ttc aac aat ctg ttc agt gaa     1140
   Met Val Met Lys His Val Gly Phe Ser Phe Asn Asn Leu Phe Ser Glu
   225                 230                 235                 240
   gcg atg gcg gta cac ccg aaa ggt gtc gac atc atg aag ccg ggc ggg     1188
   Ala Met Ala Val His Pro Lys Gly Val Asp Ile Met Lys Pro Gly Gly
                   245                 250                 255
   ctg gtg aaa gat ccg atc tcc gcg ctc tct ctg ggt ctg gga ctg atg     1236
   Leu Val Lys Asp Pro Ile Ser Ala Leu Ser Leu Gly Leu Gly Leu Met
               260                 265                 270
   ttt ggt acg gcg ggc ttg ccg cac att ctg atg cgc ttc ttt aca gtc     1284
   Phe Gly Thr Ala Gly Leu Pro His Ile Leu Met Arg Phe Phe Thr Val
           275                 280                 285
   agc gat gcc cgc gaa gca cgt aag agc gtg ttc tac gcc acc ggg ttt     1332
   Ser Asp Ala Arg Glu Ala Arg Lys Ser Val Phe Tyr Ala Thr Gly Phe
       290                 295                 300
   atg ggc tac ttc tat att ctg acc ttt att atc ggc ttc ggc gcg atc     1380
   Met Gly Tyr Phe Tyr Ile Leu Thr Phe Ile Ile Gly Phe Gly Ala Ile
   305                 310                 315                 320
   atg ctg gtt ggt gcg aat ccg gaa tat aaa gac gcg gcg ggc cat ctg     1428
   Met Leu Val Gly Ala Asn Pro Glu Tyr Lys Asp Ala Ala Gly His Leu
                   325                 330                 335
   att ggt ggt aac aac atg gcg gcc gtt cac ctg gcg aat gca gtg ggc      1476
   Ile Gly Gly Asn Asn Met Ala Ala Val His Leu Ala Asn Ala Val Gly
               340                 345                 350
   ggc aac ctg ttc ctc ggt ttt att tca gcg gtt gct ttc gcc act atc      1524
   Gly Asn Leu Phe Leu Gly Phe Ile Ser Ala Val Ala Phe Ala Thr Ile
           355                 360                 365
   ctc gcg gtg gtt gcg ggt ctg acg ctg gcg ggc gca tcc gcg gtt tcg      1572
   Leu Ala Val Val Ala Gly Leu Thr Leu Ala Gly Ala Ser Ala Val Ser
       370                 375                 380
   cat gac ttg tac gct aac gtc ttc aaa aaa ggc gcg acc gaa cgt gaa      1620
   His Asp Leu Tyr Ala Asn Val Phe Lys Lys Gly Ala Thr Glu Arg Glu
   385                 390                 395                 400
   gag ctg cgg gta tca aaa atc acc gta ctg atc ctc ggc gtg att gcg      1668
   Glu Leu Arg Val Ser Lys Ile Thr Val Leu Ile Leu Gly Val Ile Ala
                   405                 410                 415
   att atc ctc ggc gtg ctg ttt gag aat cag aac atc gcc ttt atg gtg      1716
   Ile Ile Leu Gly Val Leu Phe Glu Asn Gln Asn Ile Ala Phe Met Val
               420                 425                 430
   ggg ctg gcg ttt gcc atc gcg gcg agc tgt aac ttc ccg atc att ctg      1764
   Gly Leu Ala Phe Ala Ile Ala Ala Ser Cys Asn Phe Pro Ile Ile Leu
           435                 440                 445
   ctt tct atg tac tgg tcg aaa ctg acc acg cgt ggc gcg atg atg ggt      1812
   Leu Ser Met Tyr Trp Ser Lys Leu Thr Thr Arg Gly Ala Met Met Gly
       450                 455                 460
   ggc tgg ctg ggg ctg att acc gca gta gta ctg atg atc ctc ggc ccg      1860
   Gly Trp Leu Gly Leu Ile Thr Ala Val Val Leu Met Ile Leu Gly Pro
   465                 470                 475                 480
   acg att tgg gta cag atc ctt ggt cac gaa aaa gcc atc ttc ccg tat      1908
   Thr Ile Trp Val Gln Ile Leu Gly His Glu Lys Ala Ile Phe Pro Tyr
                   485                 490                 495
   gaa tac ccg gcg ctg ttc tct atc acc gtg gca ttc ctc ggc atc tgg      1956
   Glu Tyr Pro Ala Leu Phe Ser Ile Thr Val Ala Phe Leu Gly Ile Trp
               500                 505                 510
   ttc ttc tcg gca acc gat aac tca gcg gaa ggc gcg cgt gag cgt gaa      2004
   Phe Phe Ser Ala Thr Asp Asn Ser Ala Glu Gly Ala Arg Glu Arg Glu
           515                 520                 525
   ctg ttc cgc gcg cag ttt atc cgc tcc cag acc ggc ttt ggc gtt gag      2052
   Leu Phe Arg Ala Gln Phe Ile Arg Ser Gln Thr Gly Phe Gly Val Glu
       530                 535                 540
   caa ggc cgc gcg cat taatcgagct ctagatc                               2084
   Gln Gly Arg Ala His
   545
<210> 10
   <211> 549
   <212> PRT
   <213> Corynebacterium glutamicum
<400> 10
   Met Lys Arg Val Leu Thr Ala Leu Ala Ala Thr Leu Pro Phe Ala Ala
   1               5                   10                  15
   Asn Ala Ala Asp Ala Ile Ser Gly Ala Val Glu Arg Gln Pro Thr Asn
               20                  25                  30
   Trp Gln Ala Ile Ile Met Phe Leu Ile Phe Val Val Phe Thr Leu Gly
           35                  40                  45
   Ile Thr Tyr Trp Ala Ser Lys Arg Val Arg Ser Arg Ser Asp Tyr Tyr
       50                  55                  60
   Thr Ala Gly Gly Asn Ile Thr Gly Phe Gln Asn Gly Leu Ala Ile Ala
   65                  70                  75                  80
   Gly Asp Tyr Met Ser Ala Ala Ser Phe Leu Gly Ile Ser Ala Leu Val
                   85                  90                  95
   Phe Thr Ser Gly Tyr Asp Gly Leu Ile Tyr Ser Leu Gly Phe Leu Val
               100                 105                 110
   Gly Trp Pro Ile Ile Leu Phe Leu Ile Ala Glu Arg Leu Arg Asn Leu
           115                 120                 125
   Gly Arg Tyr Thr Phe Ala Asp Val Ala Ser Tyr Arg Leu Lys Gln Gly
       130                 135                 140
   Pro Ile Arg Ile Leu Ser Ala Cys Gly Ser Leu Val Val Val Ala Leu
   145                 150                 155                 160
   Tyr Leu Ile Ala Gln Met Val Gly Ala Gly Lys Leu Ile Glu Leu Leu
                   165                 170                 175
   Phe Gly Leu Asn Tyr His Ile Ala Val Val Leu Val Gly Val Leu Met
               180                 185                 190
   Met Met Tyr Val Leu Phe Gly Gly Met Leu Ala Thr Thr Trp Val Gln
           195                 200                 205
   Ile Ile Lys Ala Val Leu Leu Leu Phe Gly Ala Ser Phe Met Ala Phe
       210                 215                 220
   Met Val Met Lys His Val Gly Phe Ser Phe Asn Asn Leu Phe Ser Glu
   225                 230                 235                 240
   Ala Met Ala Val His Pro Lys Gly Val Asp Ile Met Lys Pro Gly Gly
                   245                 250                 255
   Leu Val Lys Asp Pro Ile Ser Ala Leu Ser Leu Gly Leu Gly Leu Met
               260                 265                 270
   Phe Gly Thr Ala Gly Leu Pro His Ile Leu Met Arg Phe Phe Thr Val
           275                 280                 285
   Ser Asp Ala Arg Glu Ala Arg Lys Ser Val Phe Tyr Ala Thr Gly Phe
       290                 295                 300
   Met Gly Tyr Phe Tyr Ile Leu Thr Phe Ile Ile Gly Phe Gly Ala Ile
   305                 310                 315                 320
   Met Leu Val Gly Ala Asn Pro Glu Tyr Lys Asp Ala Ala Gly His Leu
                   325                 330                 335
   Ile Gly Gly Asn Asn Met Ala Ala Val His Leu Ala Asn Ala Val Gly
               340                 345                 350
   Gly Asn Leu Phe Leu Gly Phe Ile Ser Ala Val Ala Phe Ala Thr Ile
           355                 360                 365
   Leu Ala Val Val Ala Gly Leu Thr Leu Ala Gly Ala Ser Ala Val Ser
       370                 375                 380
   His Asp Leu Tyr Ala Asn Val Phe Lys Lys Gly Ala Thr Glu Arg Glu
   385                 390                 395                 400
   Glu Leu Arg Val Ser Lys Ile Thr Val Leu Ile Leu Gly Val Ile Ala
                   405                 410                 415
   Ile Ile Leu Gly Val Leu Phe Glu Asn Gln Asn Ile Ala Phe Met Val
               420                 425                 430
   Gly Leu Ala Phe Ala Ile Ala Ala Ser Cys Asn Phe Pro Ile Ile Leu
           435                 440                 445
   Leu Ser Met Tyr Trp Ser Lys Leu Thr Thr Arg Gly Ala Met Met Gly
       450                 455                 460
   Gly Trp Leu Gly Leu Ile Thr Ala Val Val Leu Met Ile Leu Gly Pro
   465                 470                 475                 480
   Thr Ile Trp Val Gln Ile Leu Gly His Glu Lys Ala Ile Phe Pro Tyr
                   485                 490                 495
   Glu Tyr Pro Ala Leu Phe Ser Ile Thr Val Ala Phe Leu Gly Ile Trp
               500                 505                 510
   Phe Phe Ser Ala Thr Asp Asn Ser Ala Glu Gly Ala Arg Glu Arg Glu
           515                 520                 525
   Leu Phe Arg Ala Gln Phe Ile Arg Ser Gln Thr Gly Phe Gly Val Glu
       530                 535                 540
   Gln Gly Arg Ala His
   545